# EUROPEAN PATENT APPLICATION

(11) **EP 4 130 251 A1**
(43) Date of publication of application: **08.02.2023**
(21) Application number: 21782018.2
(22) Date of filing: 02.04.2021
(51) Int. Cl.: C12N 5/0775, A61K 35/28, A61K 35/50, A61K 35/51, A61L 27/38, A61P 43/00

(54) **HIGH-POTENTIAL PLURIPOTENT STEM CELLS**

(30) Priority: 02.04.2020 JP 2020067038; 01.12.2020 JP 2020199458
(71) Applicant: Tohoku University, Sendai-shi, Miyagi 980-8577 (JP); Life Science Institute, Inc., Tokyo 100-8251 (JP)
(72) Inventor: DEZAWA, Mari, Sendai-shi, Miyagi 980-8577 (JP); KUSHIDA, Yoshihiro, Sendai-shi, Miyagi 980-8577 (JP)
(74) Representative: Gill Jennings & Every LLP
(86) International application number: PCT/JP2021/014365
(87) International publication number: WO 2021/201286

(57) **Abstract**

The high-potential pluripotent stem cells of the present invention not only have characteristics of conventional Muse cells, namely, are capable of differentiating into any embryonic tissue serving as all cells constituting the body, but also are capable of differentiating into any extraembryonic tissue cells such as placenta and/or germline cells, namely, have differentiation capacities close to totipotency. Thus, the high-potential pluripotent stem cells not only allow for conventional regenerative therapies of various diseases which cause any damage in constitution of the body, but also are capable of differentiating into any extraembryonic tissue cells such as placenta, and thus can allow any damaged sites in such any extraembryonic tissue to be re-constituted, resulting in improvement or restoring of the function of such any damaged sites, and can be applied to a new field of regenerative therapy, for example, can be used in reproductive therapies such as fertility therapy.

## Description

### TECHNICAL FIELD

The present invention relates to a cell product in regenerative therapy. More specifically, the present invention relates to high-potential pluripotent stem cells that are isolated from extraembryonic tissue or the like, and a cell product comprising the high-potential pluripotent stem cells.

### BACKGROUND ART

Mesenchymal stem cells, one of tissue stem cells, are present in mesenchymal tissue such as bone marrow, skin, and fat, and are constituted from various cell populations. Mesenchymal stem cells have been reported to be differentiated, unlike other tissue stem cells, into not only mesodermal cells including bone, fat, and cartilage belonging to the embryologically identical system, but also ectodermal/endodermal cells beyond germ layers. While these mesenchymal stem cells can be differentiated into triploblastic-lineage cells such mesodermal, endodermal and ectodermal cells and thus are suggested to be pluripotent, these cells are low in rate of differentiation and it is thus presumed that not all of these mesenchymal stem cells, but some of these cells are pluripotent.

In view of these circumstances, the inventors have made intensive studies in order to find pluripotent stem cells considered to be present in mesenchymal stem cells, and have found new pluripotent stem cells (Multilineage-differentiating stress enduring cells: Muse cells) (Patent Document 1 and Non-patent Documents 1 to 3).

Muse cells are cells that are pluripotent so as to be differentiated into triploblastic-lineage cells, but are not tumorigenic. Muse cells have been found to home/engraft to damaged tissues only by intravenous administration, and to spontaneously differentiate into cells that comprise the tissue by following to "theory of the site", delivering tissue repair and functional recovery. For these beneficial characters, they have been studied for application to regenerative therapies for various diseases (Patent Documents 2 to 4).

It is known that Muse cells can be obtained from the bone marrow aspirate, peripheral blood, adipose tissue (Non-patent Document 4) and dermal connective tissue of the skin, and are also broadly located in organ connective tissues.

While researches of mesenchymal stem cells have been previously often made with, as raw materials, cells collected from bone marrow aspirates, adipose tissues, and the like by methods invasive to living bodies, such researches have also been recently made with, as raw materials, cells collected from medical waste such as placenta tissues (including umbilical cord, cord blood, placenta, chorion, amnion, and amniotic fluid) by methods non-invasive to living bodies (Patent Document 5).

### RELATED ART DOCUMENTS

### PATENT DOCUMENTS

Patent Document 1: Japanese Patent No. 5185443
Patent Document 2: WO2014/027684
Patent Document 3: Japanese Patent Application Publication No. 2015-159895
Patent Document 4: WO2018/235834
Patent Document 5: Japanese Translation of PCT International Application Publication No. 2020-503375

### NON-PATENT DOCUMENTS

Non-patent Document 1: Kuroda Y et al. Proc Natl Acad Sci USA, 2010: 107: 8639-8643.
Non-patent Document 2: Wakao S et al. Proc Natl Acad Sci USA, 2011: 108: 9875-9880.
Non-patent Document 3: Kuroda Y et al. Nat Protc, 2013: 8: 1391-1415.
Non-patent Document 4: Ogura F. et al., Stem Cells Dev., 23, 717-728, doi: 10.1089/scd.2013.0473 (2014)

### SUMMARY OF THE INVENTION

### PROBLEMS TO BE SOLVED BY THE INVENTION

An object of the present invention is to provide high-potential pluripotent stem cells not only having characteristics of conventional Muse cells, namely, capable of differentiating into any embryonic tissue serving as all cells constituting the body, but also capable of differentiating into any extraembryonic tissue cell such as placenta and/or germline cells, namely, having differentiation capacities close to totipotency, and also to provide a cell product comprising the high-potential pluripotent stem cells.

### MEANS FOR SOLVING THE PROBLEMS

The inventors have tried to isolate Muse cells with, as raw materials, cells that can be collected according to a non-invasive method, such as extraembryonic tissue (umbilical cord, cord blood, placenta, placenta blood, decidua, chorion, amnion, amniotic fluid, and the like) being living tissue that completes roles as organs after pregnancy-childbirth-expulsion and then serves as waste. The inventors have made detailed studies about SSEA-3-positive cells (so-called Muse cells) obtained, and thus have found that high-potential pluripotent stem cells different from Muse cells is present in the SSEA-3-positive cells obtained and have found that the high-potential pluripotent stem cells not only has useful characteristics of conventional Muse cells obtained from bone marrow and/or the like, but also has differentiation capacities into any extraembryonic tissue cells and/or germline cells and has differentiation capacities close to totipotency, thereby completed the present invention.

Accordingly, the present invention provides the following Items.
[1] SSEA-3-positive high-potential pluripotent stem cells.
[2] The SSEA-3-positive high-potential pluripotent stem cells of Item [1], wherein the pluripotent stem cell is derived from an extraembryonic tissue.
[3] The SSEA-3-positive high-potential pluripotent stem cells of Item [2], wherein the extraembryonic tissue is selected from the group consisting of umbilical cord, cord blood, placenta, placenta blood, decidua, chorion, amnion, amniotic fluid, and the like.
[4] The SSEA-3-positive high-potential pluripotent stem cells of Items [1] to [3], wherein the pluripotent stem cell is CD133-positive.
[5] The SSEA-3-positive high-potential pluripotent stem cells of Items [1] to [4], wherein the pluripotent stem cell has differentiation capacities into any extraembryonic tissue cells and/or germline cells.
[6] The SSEA-3-positive high-potential pluripotent stem cells of Items [1] to [5], wherein the pluripotent stem cell has at least one of the following characteristics:
   (i) having low or no telomerase activity;
   (ii) capable of differentiating into any of triploblastic-lineage cells as having the three germ layers;
   (iii) showing non-tumorigenic proliferation; and
   (iv) having self-renewal capacities.
[7] The SSEA-3-positive high-potential pluripotent stem cells of Items [1] to [6], wherein the pluripotent stem cell has all of the following characteristics:
   (i) having low or no telomerase activity;
   (ii) capable of differentiating into any of triploblastic-lineage cells as having the three germ layers;
   (iii) showing non-tumorigenic proliferation; and
   (iv) having self-renewal capacities.
[8] A regenerative medicine comprising the SSEA-3-positive high-potential pluripotent stem cells of Items [1] to [7].

### EFFECT OF THE INVENTION

The high-potential pluripotent stem cells of the present invention not only have characteristics of conventional Muse cells, namely, are capable of differentiating into any embryonic tissue serving as all cells constituting the body, but also is capable of differentiating into any extraembryonic tissue cells such as placenta and/or germline cells, namely, have differentiation capacities close to totipotency, and furthermore have characteristics, for example, positivity of CD133 as a cell surface marker that is negative in conventional Muse cells obtained from the bone marrow, peripheral blood, fat, skin, and/or the like. Thus, the high-potential pluripotent stem cells not only allow for conventional regenerative medicine of various diseases which cause damage of tissue constituting the body, but also are capable of differentiating into any extraembryonic tissue cell such as the placenta, and thus can allow any damaged sites in such extraembryonic tissues to be re-constituted, resulting in improvement or restoring of the function of such any damaged sites. The high-potential pluripotent stem cells are capable of differentiating into germline cells, and thus can also be used in therapies of reproductive disorders, such as fertility therapy.

Therefore, the high-potential pluripotent stem cells of the present invention and the cell product comprising the high-potential pluripotent stem cells of the present invention can be applied to a new field of regenerative therapy.

The high-potential pluripotent stem cells of the present invention can selectively migrate, and accumulate/engraft to a damaged sites, as in conventional Muse cells, and can differentiate into any tissue-constituting cell at the engrafted sites and replace multiple damaged cell/dead cell types that comprise the tissue to repair the tissue, and thus it does not require cytokine-based and/or gene introduction-based differentiation induction into target cell types prior to transplantation (administration). The high-potential pluripotent stem cells are non-tumorigenic and are thus have low safety concerns. Furthermore, since the high-potential pluripotent stem cells of the present invention do not induce any immune rejection as in conventional Muse cells, treatment with allogenic preparations produced from donors is also possible with no needs for any of HLA matching test or long-term administration of immunosuppressant. Therefore, the high-potential pluripotent stem cells of the present invention, having the excellent characteristics as described above, can provide feasible means for treatment of a patient having damage in the tissue(s) formed from an embryonic tissue and/or an extraembryonic tissue, and also for reproductive therapy. The high-potential pluripotent stem cells of the present invention allow for high expression of HLA-G, relevant to immune privilege, and are suggested to have usability such as a reduction in risks of rejection in donor preparation transplantation.

Furthermore, the high-potential pluripotent stem cells of the present invention allow for high expression of a desmosome-associated molecule, in particular, desmoglein2, desmocollin2, desmocollin3, plakoglobin, or the like, which is expressed also in the early embryo in normal development and in pluripotent state and which are considered to be essential for retention of pluripotency and self-renewal in a human pluripotent stem cell or the like, and are suggested to retain an excellent pluripotent function and have a self-renewal function and are suggested to be able to be used in a useful regenerative medicine because of resulting in more excellent improvement or restoring of the function at the damaged sites, against various diseases causing tissue damage, and furthermore of being capable of differentiating into germline cells.

### BRIEF DESCRIPTION OF THE DRAWINGS

FIG. 1 shows an isolation step of SSEA-3-positive cells (UC-HP-PSC) from human umbilical cord (including some photographs).
FIG. 2 shows the results of examination of expression of various markers in human umbilical cord-derived SSEA-3-positive cells (UC-HP-PSC).
FIG. 3 shows photographs representing an embryoid body-like clusters obtained by suspension culture of UC-HP-PSCs (human umbilical cord-derived SSEA-3-positive cells) (upper photograph), and the results of immunostaining on the embryoid body-like clusters (lower photographs).
FIG. 4 shows the results of immunostaining or RT-PCR of expression of each triploblastic-lineage markers in spontaneously differentiated cells that are expanded on gelatin-coated dishes from the embryoid body-like clusters derived from UC-HP-PSCs (human umbilical cord-derived SSEA-3-positive cells) (photographs).
FIG. 5 shows the results of differentiation of UC-HP-PSCs (human umbilical cord-derived SSEA-3-positive cells) into triploblastic-lineage cells (photographs).
FIG. 6 shows the results of RT-PCR of expression of endoderm marker, mesodermal and ectodermal markers of spontaneous differentiated cells expanded from a pluripotent cluster obtained from single cell suspension culture of UC-HP-PSCs (human umbilical cord-derived SSEA-3-positive cells) (1st cluster) , a pluripotent cluster (2nd cluster) obtained from single cell formed from 1st cluster , and a pluripotent cluster (3rd cluster) obtained from 2nd cluster on gelatin-coated dish.
FIG. 7 shows the results of the teratoma formation assay of UC-HP-PSCs (human umbilical cord-derived SSEA-3-positive cells)-transplanted SCID-mouse (photographs).
FIG. 8-1 shows cell morphology of UC-HP-PSCs (human umbilical cord-derived SSEA-3-positive cells) as well as Muse cells from other sources after trophoblast differentiation (conditions: 10 ng/mL BMP4, 1 µM A83-01, 0.1 µM PD173074) (photographs).
FIG. 8-2 shows cell morphology of UC-HP-PSCs (human umbilical cord-derived SSEA-3-positive cells) as well as Muse cells from other sources after trophoblast differentiation (conditions: 10 ng/mL BMP4, 20 µM SB431542, 20 µM SU5402) (photographs).
FIG. 8-3 shows cell morphology of UC-HP-PSCs (human umbilical cord-derived SSEA-3-positive cells) as well as Muse cells from other sources after trophoblast differentiation (photographs).
FIG. 9 shows the immunostaining of trophoblast markers in UC-HP-PSCs (human umbilical cord-derived SSEA-3-positive cells) as well as Muse cells from other sources after trophoblast differentiation (photographs).
FIG. 10-1 shows quantitative PCR of expression of trophoblast markers in UC-HP-PSCs (human umbilical cord-derived SSEA-3-positive cells) as well as Muse cells from other sources after trophoblast differentiation (conditions: 10 ng/mL BMP4, 1 µM A83-01, 0.1 µM PD173074).
FIG. 10-2 shows quantitative PCR of expression of trophoblast markers in UC-HP-PSCs (human umbilical cord-derived SSEA-3-positive cells) as well as Muse cells from other sources after trophoblast differentiation (conditions: 10 ng/mL BMP4, 20 µM SB431542, 20 µM SU5402).
FIG. 11-1 shows the results of examination of expression of CD133 in a human pluripotent embryonal carcinoma cell line NTERA-2
FIG. 11-2 shows the results of examination of expression of CD133 in a human bone marrow-derived Muse cell (BM-Muse).
FIG. 11-3 shows the results of examination of expression of CD133 in UC-HP-PSC (human umbilical cord-derived SSEA-3-positive cell).
FIG. 12-1 shows quantitative PCR of germline markers in UC-HP-PSC (human umbilical cord-derived SSEA-3-positive cell) after primordial germ cell induction, referring to the method of primordial germ cell induction in iPS cells.
FIG. 12-2 shows quantitative PCR of germline markers in human bone marrow-derived Muse cell (BM-Muse) after primordial germ cell induction, referring to the method of primordial germ cell induction in iPS cells.
FIG. 13 shows the immunostaining for Blimp 1 as a germline marker in samples fixed by 4%PFA/0.1M phosphate buffer on day 4 and day 6 after primordial germ cell induction in UC-HP-PSCs (human umbilical cord-derived SSEA-3-positive cells) and in human bone marrow-derived Muse cell (BM-Muse), referring to the method of primordial germ cell induction in iPS cells.
FIG. 14-1 shows quantitative PCR of Prdm 14, Blimp 1, Dppa3, Daz1, Nanos3, SSEA-1, Stra8, and Sycp3 as germline markers on day 3, day 5, day 7, and day 14 after primordial germ cell induction in UC-HP-PSCs (human umbilical cord-derived SSEA-3-positive cells) according to differentiation induction methods <1>, <2> and <3> with male mouse primordial germ cells.
FIG. 14-2 shows quantitative PCR of Prdm14, Blimp 1, Dppa3, Daz1, Nanos3, SSEA-1, Stra8, and Sycp3 as germline markers on day 3, day 5, day 7, and day 14 after primordial germ cell induction in UC-HP-PSCs (human umbilical cord-derived SSEA-3-positive cells) according to differentiation induction methods <1>, <2> and <3> with female mouse primordial germ cells.
FIG. 15 shows the results of immunostaining for mitochondria and Blimp 1 as a germline marker in samples fixed by 4%PFA/0.1M phosphate buffer on day 4 and day 6 after primordial germ cell induction of UC-HP-PSCs (human umbilical cord-derived SSEA-3-positive cells) according to differentiation induction methods <3> (photographs).
FIG. 16 shows a human amnion mesenchymal stem cells (AM-HP-PSC), a SSEA-3-positive cell obtained therefrom, and an embryoid body-like clusters formed in suspension culture (including some photographs).
FIG. 17 shows the results of an embryoid body-like cluster of AM-HP-PSC (human amnion-derived SSEA-3-positive cell) that was subjected to adherent culture on a gelatin-coated dish and immunostaining of endodermal, mesodermal and ectodermal markers in expanded cells from the cluster (photographs).
FIG. 18 shows the results of flow cytometry analysis of expression of CD105 or CD90 in human amnion and human bone marrow mesenchymal stem cells, and SSEA-3-positive cells (AM-HP-PSCs and BM Muse cells) respectively obtained therefrom.
FIG. 19 shows the results of flow cytometry analysis of expression of CD44 or CD133 in human amnion and human bone marrow mesenchymal stem cells, and SSEA-3-positive cells (AM-HP-PSC and BM Muse cell) respectively obtained therefrom.
FIG. 20 shows the results of flow cytometry analysis of expression of CD34 or CD45 in human amnion and human bone marrow mesenchymal stem cells, and the like.
FIG. 21 shows the comparative analysis of gene expression of germline markers in human amnion-derived SSEA-3-positive cells (AM-HP-PSCs) and a human bone marrow-derived Muse cell (BM Muse cell) using quantitative PCR.
FIG. 22 shows the results of comparative analysis of gene expression of pluripotency markers between human amnion-derived SSEA-3-positive cells (AM-HP-PSCs) and human bone marrow-derived Muse cells (BM Muse cells) using quantitative PCR.
FIG. 23 shows a preparation method of a human placenta tissue-derived high-potential pluripotent stem cells (P-HP-PSCs) (some photographs).
FIG. 24 shows the results of flow cytometry analysis of expression of CD133 in a human placenta-derived SSEA-3-positive cell.
FIG. 25-1 shows quantitative PCR of early and late germline markers in human placenta-derived high-potential pluripotent stem cells (P-HP-PSCs) that were allowed to phagocytose male mouse primordial germ cells for induction into germline cells.
FIG. 25-2 shows quantitative PCR of gene expression of early and late germline markers in human placenta-derived high-potential pluripotent stem cells (P-HP-PSCs) that were allowed to phagocytose female mouse primordial germ cells for induction into germline cells.
FIG. 26-1 shows the results of flow cytometry analysis of the proportion of a cell expressing HLA-G in a human umbilical cord-derived high-potential pluripotent stem cell fraction (UC-HP-PSC).
FIG. 26-2 shows the results of flow cytometry analysis of the proportion of a cell expressing HLA-G in a human placenta-derived high-potential pluripotent stem cell fraction (P-HP-PSC).
FIG. 26-3 shows the results of flow cytometry analysis of the proportion of a cell expressing HLA-G in a human bone marrow-derived Muse cell (BM-Muse).
FIG. 26-4 shows the results of flow cytometry analysis of the proportion of a cell expressing HLA-G in a human adipose-derived Muse cell (ADSC-Muse).
FIG. 26-5 shows the results of flow cytometry analysis of the proportion of a cell expressing HLA-G in a human skin-derived Muse cell (NHDF-Muse).
FIG. 27-1 shows the ejection fraction (EF) in a rat myocardial infarction model administrated vehicle (Control), human bone marrow-derived Muse cell (BM-Muse), human umbilical cord-derived high-potential pluripotent stem cell fraction (UC-HP-PSC) and human bone marrow-derived SSEA-3 negative MSC (BM non-Muse).
FIG. 27-2 shows the change in ejection fraction (ΔEF) in a rat myocardial infarction model treated with vehicle (Control), a human bone marrow-derived Muse cell (BM-Muse), a human umbilical cord-derived high-potential pluripotent stem cell fraction (UC-HP-PSC) or a human bone marrow-derived SSEA-3 negative MSC (BM non-Muse).
FIG. 27-3 shows the left ventricular fractional shortening (%FS) in a rat myocardial infarction model treated with vehicle (Control), human bone marrow-derived Muse cell (BM-Muse), human umbilical cord-derived high-potential pluripotent stem cell fraction (UC-HP-PSC) and human bone marrow-derived SSEA-3 negative MSC (BM non-Muse).
FIG. 27-4 shows the change in left ventricular fractional shortening (Δ%FS) in a rat myocardial infarction model treated with vehicle (Control), human bone marrow-derived Muse cell (BM-Muse), human umbilical cord-derived high-potential pluripotent stem cell fraction (UC-HP-PSC), and human bone marrow-derived SSEA-3 negative MSC (BM non-Muse).
FIG. 28 shows the results of gene expression of desmosome-associated molecules in a human umbilical cord-derived high-potential pluripotent stem cells (UC-HP-PSC), a human bone marrow-derived Muse cell (BM-Muse), a human adipose-derived Muse cell (ADSC-Muse), and a human skin-derived Muse cell (NHDF-Muse) using RNA-seq. The vertical axis represents the number of fragments per kilobase of exon per million mapped reads (FPKM).

### DETAILED DESCRIPTION OF THE INVENTION

The present invention relates to a high-potential pluripotent stem cells that are isolated from extraembryonic tissue or the like, and a cell product comprising the high-potential pluripotent stem cells. The present invention will be described in detail below.

### 1. Cell product

### (1) High-potential pluripotent stem cells

The high-potential pluripotent stem cells to be used in the cell product of the present invention are high-potential pluripotent stem cells that are isolated from extraembryonic tissues or the like, and the stem cells have useful characteristics of pluripotent stem cells as in Muse cells described below, additionally have differentiation capacities into any extraembryonic tissue cells and/or germline cells and has differentiation capacities close to totipotency, and furthermore has characteristics, for example, positivity for CD133 as a cell surface marker that is negative in conventional Muse cells obtained from the bone marrow, peripheral blood, fat, skin, or the like.

In particular, as shown below, a human umbilical cord-derived SSEA-3-positive cells obtained in Example 1 have useful characteristics of pluripotent stem cells as in conventional Muse cells obtained from the bone marrow, peripheral blood, fat, skin, and/or the like, additionally have differentiation capacities into any extraembryonic tissue cells and/or germline cells and have differentiation capacities close to totipotency, and furthermore have characteristics, for example, positivity for CD133 as a cell surface marker, at 99.9% or more, according to other analysis in Example 1. Thus, the human umbilical cord-derived SSEA-3-positive cells obtained in Example 1 may be referred to as "human umbilical cord-derived high-potential pluripotent stem cells," or may be abbreviated as "UC-HP-PSC" (Umbilical cord high potential pluripotent stem cells).

A human amnion-derived SSEA-3-positive cells obtained in Example 2 have useful characteristics of pluripotent stem cells as in conventional Muse cells obtained from the bone marrow, peripheral blood, fat, skin, and/or the like, additionally have differentiation capacities into any extraembryonic tissue cells and/or germline cells and have differentiation capacities close to totipotency, and furthermore have characteristics, for example, positivity for CD133 as a cell surface marker, at about 70% or more, according to other analysis. Thus, the human amnion-derived SSEA-3-positive cells obtained in Example 2 may be referred to as "human amnion-derived high-potential pluripotent stem cells" or "human amnion-derived high-potential pluripotent stem cell fraction," or may be abbreviated as "AM-HP-PSC" (Amniotic membrane High potential Pluripotent stem cells).

Furthermore, a human placenta-derived SSEA-3-positive cells obtained in Example 3 has useful characteristics of pluripotent stem cells as in conventional Muse cells obtained from the bone marrow, peripheral blood, fat, skin, and/or the like, additionally have differentiation capacities into any extraembryonic tissue cells and/or germline cells and have differentiation capacities close to totipotency, and furthermore have characteristics, for example, positivity for CD133 as a surface marker, at about 80% or more, according to other analysis. Thus, the human placenta-derived SSEA-3-positive cells obtained in Example 3 may be referred to as "human placenta-derived high-potential pluripotent stem cells" or "human placenta-derived high-potential pluripotent stem cell fraction," or may be abbreviated as "P-HP-PSC" (placenta high-potential pluripotent stem cells).

It has been confirmed that a very low percentage (up to about several percent) of the high-potential pluripotent stem cells of the present invention is present among conventional bone marrow-derived SSEA-3-positive cells. Therefore, the high-potential pluripotent stem cells of the present invention can be obtained not only from extraembryonic tissue, but also from conventional tissues such as the bone marrow, peripheral blood, fat, skin, and/or the like.

Therefore, the high-potential pluripotent stem cells of the present invention can be viewed as some special Muse cells mainly isolated from extraembryonic tissue. In other words, the high-potential pluripotent stem cells of the present invention have useful characteristics of pluripotent stem cells as in conventional Muse cells, and the characteristics means characteristics or the like of Muse cells, described below.

It is known that Muse cells can be obtained from, for example, bone marrow aspirates, adipose tissues (Non-patent Document 4), and dermal connective tissues of skin, and are also broadly present in organ connective tissues as well as in the peripheral blood. These cells also have both characteristics of pluripotent stem cells and mesenchymal stem cells and are identified as, for example, a cell positive for "SSEA-3," a cell surface marker, preferably as a double-positive for both SSEA-3 and CD105. Therefore, Muse cells or a cell population containing Muse cells can be isolated from living tissues using, for example, expression of SSEA-3 only or a combination of SSEA-3 and CD105 as an index. Methods for separation, identification and characterization of Muse cells have been disclosed in WO2011/007900. Taking advantage of the high resistance of Muse cells to various external stresses, Muse cells can be selectively enriched by culturing the cells under various external stress conditions, such as under protease treatment, under hypoxic conditions, under low phosphate conditions, in a low serum concentration, under undernutrition conditions, under heat shock exposure, in the presence of toxic substances, in the presence of reactive oxygen species, under mechanical stimulation, and under pressure treatment. As used herein, pluripotent stem cells prepared from mesenchymal tissues in a living body or cultured mesenchymal tissues using SSEA-3 as an index (Muse cells), or a cell population comprising Muse cells may be simply referred to as "SSEA-3-positive cells."

Muse cells or a cell population comprising Muse cells can be prepared from living tissues (e.g., mesenchymal tissues) using cell surface markers, SSEA-3, or SSEA-3 and CD105, as indexes. As used herein, the term "living" body means mammal living body. In the present invention, living bodies exclude fertilized egg and embryos in developmental stages before blastula stage, but include embryos in developmental stages of blastula stage or later, including fetus and blastula. Examples of the mammal include, but not limited to, primates such as human and monkey; rodents such as mouse, rat, rabbit, and guinea pig; and cat, dog, sheep, pig, cattle, horse, donkey, goat, and ferret. Muse cells to be used in the cell product of the present invention are directly isolated from living tissues using markers, and thus are clearly distinguished from embryonic stem cells (ES cells) and iPS cells. The term "mesenchymal tissue" refers to tissue such as the bone, synovial membrane, fat, blood, bone marrow, skeletal muscle, dermis, ligament, tendon, dental pulp, umbilical cord, cord blood, and amnion, as well as tissues present in various organs. For example, Muse cells can be obtained from the bone marrow, skin, adipose tissues, blood, dental pulp, umbilical cord, cord blood, or amnion and the like. For example, a mesenchymal tissue in a living body is preferably collected, and then Muse cells are prepared from the tissue. Alternatively, using the preparation method described above, Muse cells may be prepared from cultured mesenchymal cells such as fibroblasts or bone marrow mesenchymal stem cells.

The cell population containing Muse cells can be prepared by a method comprising stimulating a mesenchymal tissue in a living body or cultured mesenchymal cells with an external stress to selectively increase cells that are resistant to the external stress, and collecting the cells with an increased abundance ratio.

The external stress may be any one of or a combination of the following: protease treatment, culturing under low oxygen concentration, culturing under low phosphate conditions, culturing under low serum concentration, culturing undernutrition conditions, culturing under heat shock exposure, culturing at low temperatures, freezing treatment, culturing in the presence of toxic substances, culturing in the presence of reactive oxygen species, culturing under mechanical stimulation, culturing under shaking, culturing under pressure treatment or physical shocks.

The protease treatment is preferably carried out for 0.5 to 36 hours in total to exert an external stress. The concentration of the protease is preferably used when cells adhered to a culture vessel are peeled off, when cell aggregates are isolated into single cells, or when single cells are collected from a tissue.

Preferably, the protease is a serine protease, an aspartic protease, a cysteine protease, a metalloprotease, a glutamic protease, or an N-terminal threonine protease. More preferably, the protease is trypsin, collagenase, or dispase.

Muse cells may be autologous or allogeneic to a recipient who will receive the cells.

As described above, Muse cells or a cell population comprising Muse cells can be prepared from living tissues, for example, by using SSEA-3 positivity or SSEA-3 and CD105 double positivity as an index.

Human adult skin is known to comprise various types of stem cells and progenitor cells, and these stem cells and progenitor cells include skin-derived progenitor cells (SKP), neural crest stem cells (NCSC), melanoblasts (MB), pericytes (PC), endothelial progenitor cells (EP), and adipose-derived stem cells (ADSC).

Muse cells are not the same as these cells, and thus Muse cells can be prepared using "non-expression" of markers unique to these cells as an index. More specifically, Muse cells can be isolated using as an index non-expression of at least one, e.g., 2, 3, 4, 5, 6, 7, 8, 9, 10, or 11, of 11 markers selected from the group consisting of CD34 (a marker for EP and ADSC), CD117 (c-kit) (a marker for MB), CD146 (a marker for PC and ADSC), CD271 (NGFR) (a marker for NCSC), NG2 (a marker for PC), vWF factor (von Willebrand factor) (a marker for EP), Sox10 (a marker for NCSC), Snai1 (a marker for SKP), Slug (a marker for SKP), Tyrp1 (a marker for MB), and Dct (a marker for MB). Muse cells can be prepared by using as an index, non-expression of, for example, without limitation, CD117 and CD146; CD117, CD146, NG2, CD34, vWF, and CD271; or the above-described 11 markers.

Muse cells having the above-described characteristics may also have at least one selected from the group consisting of the following characteristics:
(i) having low or no telomerase activity;
(ii) capable of differentiating into any of triploblastic-lineage cells as having the three germ layers;
(iii) showing non-tumorigenic proliferation; and
(iv) having self-renewal capacities
Preferably, Muse cells used in the present invention have all of the characteristics described above.

With respect to (i) above, the phrase "having low or no telomerase activity" means that the telomerase activity is low or undetectable when detected using, for example, TRAPEZE XL telomerase detection kit (Millipore Corporation). Having "low" telomerase activity means, for example, having a telomerase activity comparable to somatic human fibroblast, or having 1/5 or less telomerase activity, preferably 1/10 or less telomerase activity, as compared with that of HeLa cell.

With respect to (ii) above, Muse cells are capable of being differentiated into triploblastic-lineage cells (endodermal, mesodermal, and ectodermal cells) in vitro and in vivo, and can be differentiated into, for example, hepatocytes (including cells expressing markers of hepatoblast or hepatocyte), neurons, skeletal muscle cells, smooth muscle cells, osteocytes, or adipocytes by in vitro induction culture medium. Muse cells may also show the ability to be differentiated into triploblastic-lineage cells when administrated in testis in vivo. Further, Muse cells are capable of migrating and engrafting to injured organs (such as the heart, skin, spinal cord, liver, and muscle) when administrated into a living body via intravenous injection and being differentiated into cells that are compatible to the engrafted tissue.

With respect to (iii) above, Muse cells are characterized in that they proliferate at a growth rate of about 1.3 days and proliferate from a single cell in suspension culture to form embryoid body-like clusters, and then arrest their proliferation after about 14 days when the clusters reach a certain size. When these embryoid body-like clusters are transferred to adherent culture, the cells restart proliferation and cells proliferated from the clusters expand at a growth rate of about 1.3 days. Further, Muse cells are characterized in that, when transplanted into testis, they do not become tumorigenic for at least half a year.

With respect to (iv) above, Muse cells have self-renewal (self-replication) capacities. The term "self-renewal," as used herein, means that the followings can be observed: differentiation into triploblastic-lineage cells in transfer of cells contained in first generation embryoid body-like clusters obtained by culturing single Muse cells in a suspension culture, to adherent culture; formation of second-generation embryoid body-like clusters by again culturing single cells in the first generation embryoid body-like clusters in a suspension culture, and differentiation again into triploblastic-lineage cells in transfer to adherent culture; as well as formation again of third generation embryoid body-like clusters by culturing single cells in a suspension culture. Self-renewal may be repeated for one or more cycles.

The high-potential pluripotent stem cells to be used in the cell product of the present invention is high-potential pluripotent stem cells that is isolated from extraembryonic tissue or the like, and the stem cell has useful characteristics of pluripotent stem cells as in Muse cells described above, additionally has differentiation capacities into any extraembryonic tissue cells and/or germline cells and has differentiation capacities close to totipotency, and furthermore has characteristics, for example, positivity for CD133 as a cell surface marker that is negative in conventional Muse cells obtained from the bone marrow, peripheral blood, fat, skin, or the like.

CD133 is one of the glycoproteins, and is a cell surface marker also otherwise known as Prominin 1. Examples of human CD133 include proteins recorded as accession numbers of AER93377 and AER93376 in the Protein database in NCBI (National Center for Biotechnology Information).

The term "CD133-positive" means that a cell is stained in the case of cell staining with a CD133 antibody or a cell is sorted with a CD133 antibody according to flow cytometry.

The high-potential pluripotent stem cells of the present invention can be a cell population comprising CD133-positive cells, and the cell population preferably comprises 50% or more, more preferably 60% or more, 70% or 80% or more, further preferably 90% or more of CD133-positive cells.

A cell surface marker CD133 being positive can be confirmed by an identification method with a usual antibody or the like.

The high-potential pluripotent stem cells of the present invention can be obtained from extraembryonic tissue or the like. The extraembryonic tissue or the like can be obtained from living tissue that completes roles as organs after pregnancy-childbirth-expulsion and serves as waste, such as umbilical cord, cord blood, placenta, placenta blood, decidua, chorion, amnion, and amniotic fluid and the like.

The high-potential pluripotent stem cells of the present invention not only can be differentiated into all triploblastic-lineage cells (endodermal, mesodermal, and ectodermal cells) constituting an individual, but also has differentiation capacities into any extraembryonic tissue cells and/or germline cells and has differentiation capacities close to totipotency.

The phrase "having differentiation capacities into any extraembryonic tissue cells and/or germline cells and having differentiation capacities close to totipotency" means capacities of differentiation into placenta or the like as extraembryonic tissue, gamete (sperm or egg) as germline cells, and various cells including primordial germ cells (PGC) serving as bases thereof.

Specifically, the differentiation capacities into any extraembryonic tissue cells and/or germline cells can be confirmed by, for example, differentiation into various cells as extraembryonic tissues, differentiation into trophoblast lineage, cell multinuclearity, expression of trophoblast markers (e.g., ERVW-1, human chorionic gonadotropin alpha chain (hCGA), and the like, CDX2, TP63, ID2, and the like as Early Makers, and GCM1, PGF, ERVFRD-1, and the like as Late makers), expression of genes involved in differentiation of germline cells (e.g., Blimp1, Dappa3, ITGA, Sycp3, TBX3, TFAP2c, TP63, Nanos3, PRDM14, SSEA-1, Daz1, and Stra8), and/or expression of genes exhibiting pluripotency (e.g., Oct3/4, Nanog, Sox2, KLF2, KLF4, REX1, and TERT).

In particular, the high-potential pluripotent stem cells of the present invention can be obtained by a conventional method of isolation of Muse cells from extraembryonic tissue or the like (WO2011/007900).

For example, the high-potential pluripotent stem cells of the present invention can be isolated by slicing extraembryonic tissue or the like and culturing a tissue section, culturing an extraembryonic tissue-derived mesenchymal cell for proliferation until an effective amount of cell is achieved, and then adopting (i) SSEA-3 only, (ii) SSEA-3/CD105 double, (iii) SSEA-3/CD133 double, or (iv) SSEA-3/CD105/CD133 triple antigen marker as an index.

The high-potential pluripotent stem cells of the present invention can be obtained from embryonic tissue such as bone marrow, peripheral blood, fat, and skin, according to a method of isolation from the extraembryonic tissue or the like.

The high-potential pluripotent stem cells of the present invention being a cell having differentiation capacities close to totipotency can also be confirmed by, for example, examining characteristics of a naïve or primed pluripotent stem cell shown in Table 1 below.

**Table 1: Characteristics of Naive and Primed pluripotent stem cells**

| | Naïve | Primed |
|---|---|---|
| Colony morphology | Dome type (3D) | Flat |
| Developmental stage | Pre-implantation embryo | Pro-implantation embryo |
| Reagents for maintenance medium | LIF: MEK inhibitor, GSK inhibitor | FGF. Activin |
| Chimeric formation capacities | Presence | Absence |
| Teratoma formation capacities | Presence | Presence |
| Differentiation capacities into triploblastic-lineage cells | Differentiation into triploblastic-lineage cells | Differentiation into triploblastic-lineage cells (high bias) |
| Differentiation capacities into germ cell | Presence | Absence |
| Differentiation capacities into extraembryonic cell | Presence | Absence |
| Pluripotent gene | Expression of Oct3/4, Nanog, and Sox2 | Expression of Oct3/4, Nanog, and Sox2 |
| Naive-associated gene | Expression of TFCP2L 1 and the like | No expression |
| Energy metabolism | Aerobic | Glycolytic |
| Chromosomal DNA methylation | Low level | High level |
| X-chromosome transcriptional activity | Both two | Only one |
| Transcriptional repression histone modification | Low level | High level |

### <References:

1) L. Weinberger, M. Ayyash, N. Novershtern, J. H. Hanna, Dynamic stem cell states: naive to primed pluripotency in rodents and humans. Nat. Rev. Mol. Cell Biol. 17, 155-169 (2016).
2) M. Ueda, Y. Takashima, History of Pluripotent Stem Cells and Human Naive Pluripotent Stem Cells. Cytometry Research 27, 19-24 (2017).
3) Kiichiro Tomoda, Biochemistry, vol. 90, No. 2, p. 187-191 (2018)>

Furthermore, the high-potential pluripotent stem cells of the present invention can be confirmed by expression of a desmosome-associated molecule, in particular, desmoglein2, desmocollin2, desmocollin3, plakoglobin, or the like, which is expressed also in the early embryo in normal development and pluripotent stem cells and which is considered to be essential for retention of pluripotency and self-renewal in a human pluripotent stem cells or the like.

### (2) Preparation of Cell Product

The cell product of the present invention can be obtained by, without limitation, suspending the high-potential pluripotent stem cells of the present invention or a cell population comprising the cell, obtained in (1) above, in a physiological saline or a suitable buffer solution (e.g., a phosphate buffered saline). In this case, when only a small number of the high-potential pluripotent stem cells of the present invention is isolated from an autologous or allogeneic tissue, the cell may be cultured before cell transplantation until the predetermined number of cells is attained. The high-potential pluripotent stem cells of the present invention is non-tumorigenic, and thus is less likely to be tumorigenic and thus is with low safety concerns, even if such a cell collected from a living tissue is contained in an undifferentiated state. The collected high-potential pluripotent stem cells of the present invention can be cultured in any conventional growth medium (e.g., alpha-minimum essential medium (a-MEM) supplemented with 10% fetal bovine serum), without particularly limitation. More specifically, with reference to the above-described WO2011/007900, the high-potential pluripotent stem cells of the present invention can be cultured and proliferated using appropriately selected culture medium, additives (e.g., antibiotics, and serum) and the like, to prepare a solution containing the high-potential pluripotent stem cells of the present invention at a predetermined concentration. In the case that the cell product of the present invention is administered to a human subject, first, human extraembryonic tissue is collected. Then, for example, an umbilical cord tissue-derived mesenchymal stem cells are cultured as an adherent cell obtained from umbilical cord tissue and proliferated until reaching the cell amount where a therapeutically effective amount of the high-potential pluripotent stem cells of the present invention can be obtained. Thereafter, the high-potential pluripotent stem cells of the present invention are isolated using an antigenic marker SSEA-3 as an index to prepare a cell product comprising the autologous or allogeneic high-potential pluripotent stem cells of the present invention. Alternatively, for example, extraembryonic tissue-derived mesenchymal stem cells obtained from a human extraembryonic tissue can be cultured under external stress conditions, so that the high-potential pluripotent stem cells of the present invention can be grown and enriched until the amount thereof reaches a therapeutically effective amount, thereby preparing a cell product comprising the autologous or allogeneic high-potential pluripotent stem cells of the present invention.

When the high-potential pluripotent stem cells of the present invention are used in a cell product, the cell product may also comprise dimethyl sulfoxide (DMSO), serum albumin and the like for protection of the cell, and any antibiotic and the like for prevention of contamination and proliferation of bacteria. The cell product may further comprise other pharmaceutically acceptable components (e.g., carriers, excipients, disintegrants, buffer agents, emulsifiers, suspending agents, soothing agents, stabilizers, preservatives, antiseptics, physiological saline). These agents and drugs can be added to the cell product at appropriate concentrations by the skilled person. Thus, the high-potential pluripotent stem cells of the present invention can also be used as a pharmaceutical composition comprising various additives. The dosage form of the cell product is preferably a product for parenteral administration, further preferably an injection product, without particularly limitation.

The number of such high-potential pluripotent stem cells of the present invention, comprised in the cell product prepared above, can be appropriately adjusted to achieve desired effects on a disease to be treated, in consideration of, for example, the sex, age, and weight of the subject, the condition of the affected area, and the condition of such cells to be used. Individuals as the subject include, but not limited to, mammals such as human. The cell product of the present invention may be administered in a single dose or multiple times (e.g., 2 to 10 times) at appropriate intervals (e.g., twice a day, once a day, twice a week, once a week, once every two weeks, once a month, once every two months, once every three months, or once every six months) until the desired therapeutic effect is obtained. The timing of administration may be any of an acute phase, a subacute phase, and a chronic phase as long as the therapeutic effect is obtained, and may be two or more phases selected from these phases.

The therapeutically effective amount is preferably, for example, 1 to 10 doses of 1 × 10³ to 1 × 10¹⁰ cells/individual/dose, depending on the state of the subject. The total amount administered to an individual is, without limitation, 1 × 10³ to 1 × 10¹¹ cells, preferably 1 × 10⁴ to 1 × 10¹⁰ cells, more preferably 1 × 10⁵ to 1 × 10⁹ cells.

The high-potential pluripotent stem cells of the present invention, to be used in the cell product of the present invention, are characterized in that it migrates and engrafts to sites damaged due to a disease to be treated. Thus, the sites and method of administration of the cell product in administration of the cell product are not limited, and the cell product may be administered locally to the affected area, or may be administered intravenously.

The cell product of the present invention allows for re-constituting the damaged sites in a patient having a disease to be treated, and allows for improvement or restoring of lost functions of the damaged sites in the patient.

The present invention will be described in more detail with reference to examples below, but is not limited to the examples.

### EXAMPLES

### Example 1-1: Isolation of human umbilical cord-derived high-potential pluripotent stem cells

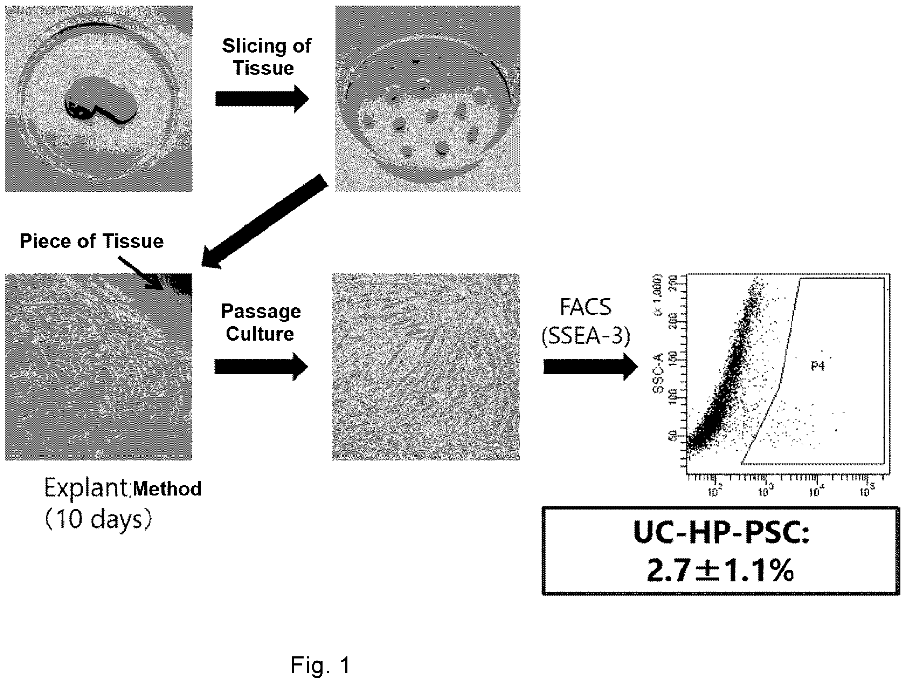

A high-potential pluripotent stem cells can be obtained from human umbilical cord according to the method for isolation and identification of human Muse cells described in WO2011/007900. More specifically, a human umbilical cord-derived high-potential pluripotent stem cells were obtained by FACS isolation with SSEA-3 as an index according to a process in FIG. 1.

Specifically, 35-week human umbilical cord was sliced to a size of 0.5-mm square, and each tissue section was placed on a 10-cm dish at a 1-cm interval. αMEM, 10% FBS, 1 ng/mL bFGF, and 2 mM GlutaMAX were added thereto, and the resultant was subjected to culture for about 10 days according to an explant method. After established human umbilical cord-derived mesenchymal stem cells were expansively/continuously cultured, a human umbilical cord tissue-derived SSEA-3-positive cells (namely, human umbilical cord-derived high-potential pluripotent stem cells of the present invention: UC-HP-PSC) was isolated with an anti-SSEA-3 antibody. About 3% of the SSEA-3-positive cell was present in the established human umbilical cord-derived mesenchymal stem cells.

### Example 1-2: Human umbilical cord-derived high-potential pluripotent stem cells co-expresses mesenchymal cell surface markers

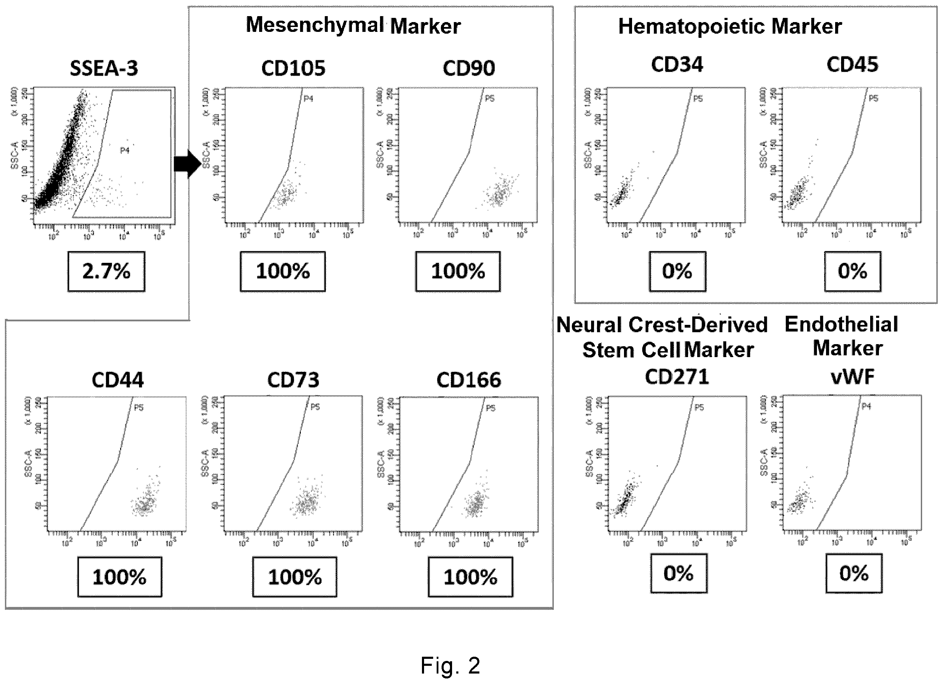

A cell surface marker of the human umbilical cord-derived SSEA-3-positive cell obtained in Example 1-1 was analyzed by FACS. As a result, the human umbilical cord-derived SSEA-3-positive cells co-expressed CD105, CD90, CD44, CD73, and CD166 as mesenchymal stem cell markers, as shown in FIG. 2. However, CD34 and CD45 as hematopoietic markers, CD271 as a neural crest-derived stem cell marker, and a von Willebrand factor (vWF) as an endothelial progenitor marker were negative.

Example 1-3: Human umbilical cord-derived high-potential pluripotent stem cells has the following characteristics as in conventional human bone marrow-derived Muse cells.

### (1) Human umbilical cord-derived high-potential pluripotent stem cells form pluripotent cluster

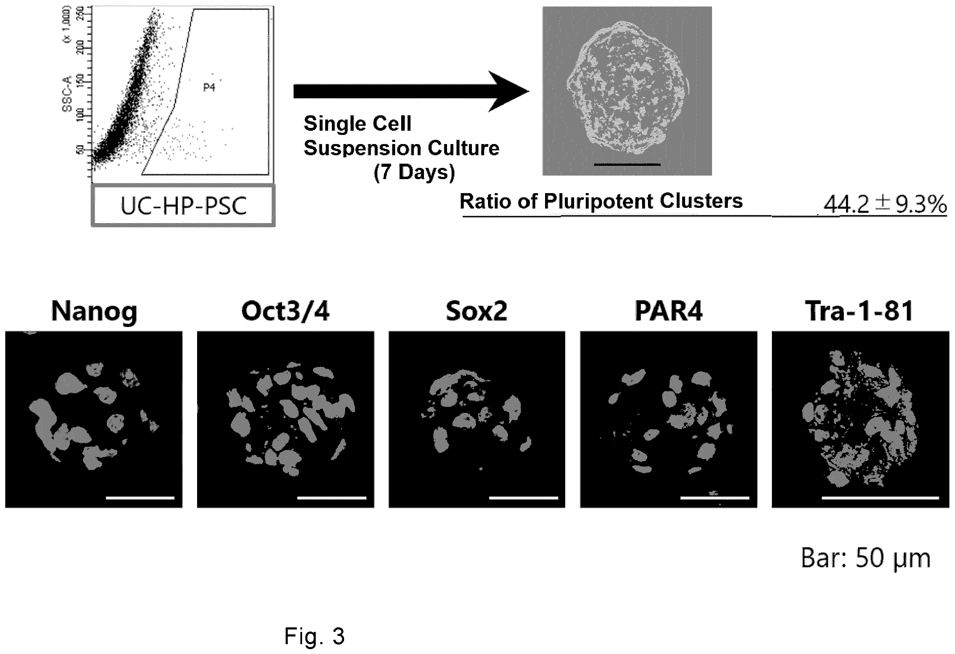

The human umbilical cord-derived SSEA-3-positive cells obtained by FACS in Example 1-1 were used and subjected to single cell suspension culture. On day 7 of the culture, 44.2% of the SSEA-3-positive cell formed embryoid body-like clusters (the upper in FIG. 3). The clusters were frozen and embedded, and sectioned, and subjected to immunostaining on Nanog, Oct3/4, Sox2, PAR4, and Tra-1-81 as pluripotency markers. As a result, the embryoid body-like clusters expressed all the pluripotency markers, and it was confirmed that the embryoid body-like clusters formed from the human umbilical cord-derived SSEA-3-positive cells were a pluripotent clusters, as shown in the lower in FIG. 3.

### (2) Human umbilical cord-derived high-potential pluripotent stem cells spontaneously differentiate into triploblastic-lineage cells

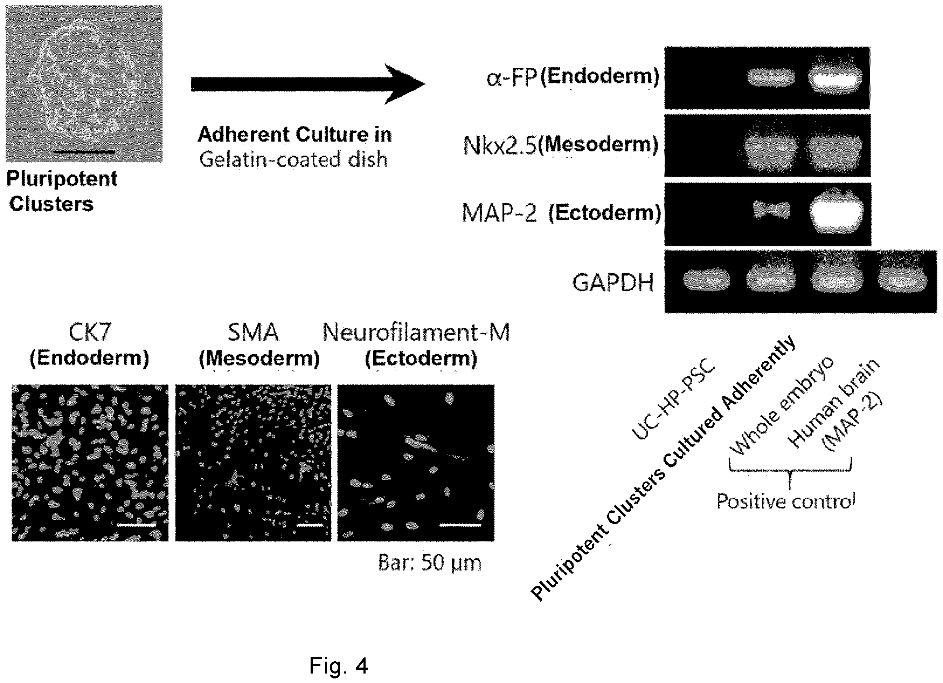

In order to confirm differentiation capacities into triploblastic-lineage cells, pluripotent clusters formed from a single human umbilical cord-derived SSEA-3-positive cell were transferred to a dish coated with gelatin, and subjected to adherent culture. After about 10 days, immunostaining was performed, and thus cytokeratin7 (CK7, endoderm marker), α-smooth muscle actin (SMA, mesoderm marker), and neurofilament-M (ectoderm marker) were detected, as shown in FIG. 4. RT-PCR was performed, and it was thus confirmed that α-fetoprotein (α-FP, endoderm marker), Nkx2.5 (mesoderm marker), and microtubule-associated protein (MAP-2, ectoderm marker) were expressed, as shown in FIG. 4. It was found from the results that the human umbilical cord-derived SSEA-3-positive cells had spontaneous differentiation capacities into triploblastic-lineage cells.

### (3) Human umbilical cord-derived high-potential pluripotent stem cells differentiate into triploblastic-lineage cells by induction of differentiation

For neural induction, the human umbilical cord-derived SSEA-3-positive cells were cultured at a density of 1 × 10⁵ cells/mL in a B-27 supplement-containing Neurobasal medium (Gibco) with a dish coated with poly-HEMA for 7 days, and spheres (spherical cell aggregates) were formed.

For osteocyte induction, the human umbilical cord-derived SSEA-3-positive cells were cultured at a density of 4 × 10³ cells/cm² with an osteocyte induction medium of Human Mesenchymal Stem Cell Functional Identification Kit (R&D Systems) for 16 days.

For adipocyte induction, the human umbilical cord-derived SSEA-3-positive cells were cultured at a density of 1 × 10⁴ cells/cm² with an adipocyte induction medium of Human Mesenchymal Stem Cell Functional Identification Kit (R&D Systems) for 16 days.

For hepatocyte induction, the human umbilical cord-derived SSEA-3-positive cells were seeded at a density of 1 × 10⁴ cells/cm² on a plate coated with collagen. After seeding, the cells were cultured with DMEM-low glucose, 20 ng/mL EGF, and 10 ng/mL bFGF for two days. Thereafter, the cells were cultured with DMEM-low glucose, 0.5 mg/mL BSA, 1 × insulin-transferrin-selenium, 5 mM nicotinamide, 10 nM dexamethasone, 150 ng/mL HGF, and 50 ng/mL FGF4 for 28 days.

For vascular endothelial cell induction, the human umbilical cord-derived SSEA-3-positive cells were cultured at a density of 5 × 10³ cells/cm² with DMEM-low-glucose, 2% FBS, and 20 ng/mL bFGF for 8 days.

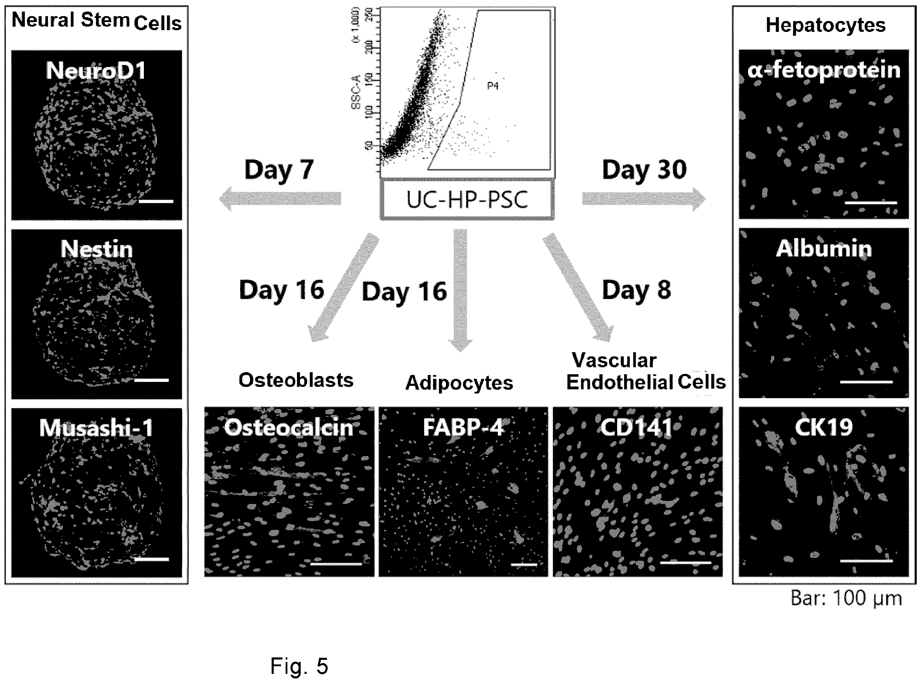

The results are shown in FIG. 5.

It was found that spheres were formed due to neural induction and the sphere was positive for NeuroD1, Nestin, and Musashi-1 as neural stem cell markers.

Expression of Osteocalcin, FABP-4, and CD141 respectively due to osteocyte induction, adipocyte induction, and vascular endothelial cell induction was observed.

Expression of α-fetoprotein, Albumin, and cytokeratin19 (CK19) due to hepatocyte induction was confirmed.

These results indicate that the human umbilical cord-derived SSEA-3-positive cells have differentiation capacities into triploblastic-lineage cells also by differentiation induction.

### (4) Human umbilical cord-derived high-potential pluripotent stem cells have self-renewal capacities

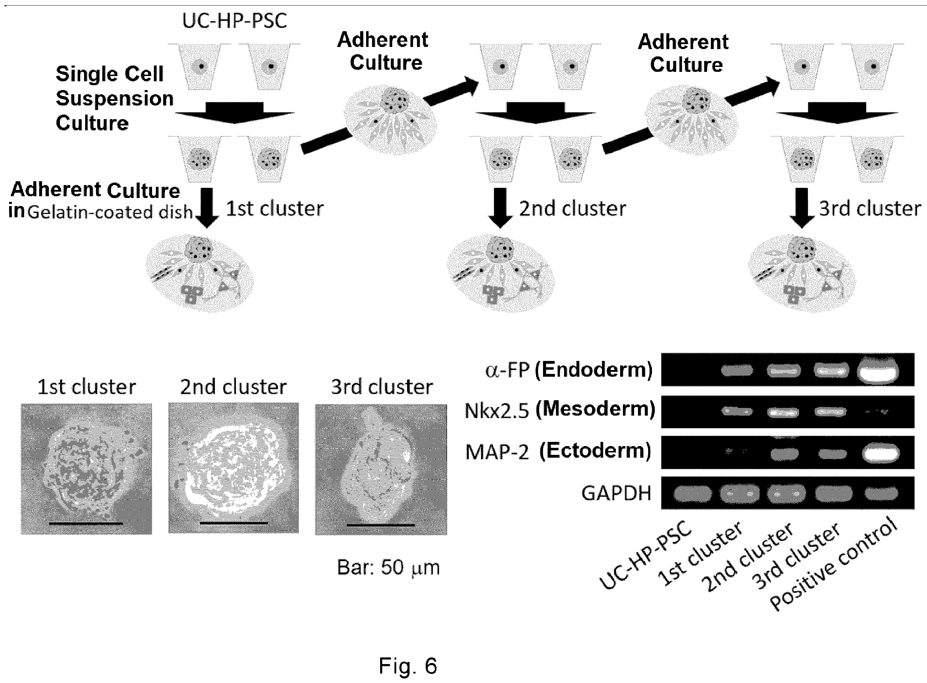

The self-renewal capacities of the human umbilical cord-derived SSEA-3-positive cells were tested with the differentiation capacities into triploblastic-lineage cells, as an index. After pluripotent clusters were formed from a single human umbilical cord-derived SSEA-3-positive cell, a single cluster was subjected to adherent culture on a dish uncoated or coated with gelatin. The cluster subjected to adherent culture on the dish uncoated was cultured for 7 days, and treated by trypsin for 5 minutes to thereby provide a single cell, single cell suspension culture was again performed, and thus a pluripotent cluster was formed (2nd cluster). On the other hand, the cluster subjected to adherent culture on the dish coated with gelatin was subjected to adherent culture for about 10 days, and thereafter RNA was extracted. The suspension culture-adherent culture was repeated three times, and it was thus confirmed that α-fetoprotein (α-FP, endoderm marker), Nkx2.5 (mesoderm marker), and microtubule-associated protein (MAP-2, ectoderm marker) were expressed in each generation (FIG. 6). It is thus found that the human umbilical cord-derived SSEA-3-positive cell has self-replication capacities and is clonally proliferated.

### (5) Human umbilical cord-derived high-potential pluripotent stem cells form no tumor even if administrated into living body

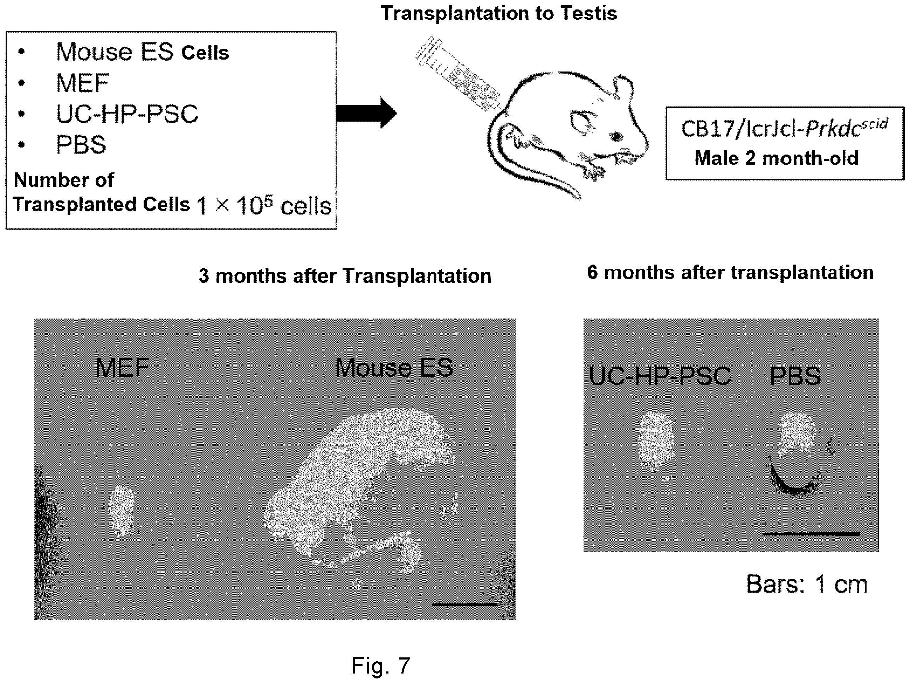

In order to evaluate the tumorigenic capacities of the human umbilical cord-derived SSEA-3-positive cells in a living body, the human umbilical cord-derived SSEA-3-positive cells were transplanted at 1 × 10⁵ cells into the testis of CB17/IcrJcl-Prkdc^{scid} (male, 2-month) as an immunodeficient mouse, by use of a glass micro tube. Mouse ES cells (positive control) at 1 × 10⁵ cells, mitomycin C treated MEF (mouse embryonic fibroblast) at 1 × 10⁵ cells and PBS (negative control) as control groups were administrated. As a result, as shown in FIG. 7, mouse ES cells formed teratoma at three months after the transplantation, whereas the human umbilical cord-derived SSEA-3-positive cells formed no teratoma even at six months after the transplantation. It was thus found that the human umbilical cord-derived SSEA-3-positive cells had no tumorigenic capacities.

Example 1-4: human umbilical cord tissue-derived high-potential pluripotent stem cells have the following characteristics not exhibited by conventional bone marrow-, adipose-, and skin-derived Muse cells, and the like

### (1) Induction of differentiation into trophoblast lineage

Differentiation induction of the human umbilical cord-derived SSEA-3-positive cells obtained in Example 1-1 into trophoblast lineage was confirmed by the following two induction methods, according to the following differentiation induction conditions for induction of differentiation of ES and iPS cells into trophoblast lineage. Human bone marrow- (BM-Muse), human adipose- (ADSC-Muse), human skin-derived (NHDF-Muse) Muse cells were used as control groups. These cells were seeded at a density of 1 × 10⁴ cells/cm² on dishes, [1] DMEM-low-glucose, 2% FBS, 10 ng/mL BMP4, 1 µM A83-01, and 0.1 µM PD173074, and [2] DMEM-low-glucose, 2% FBS, 10 ng/mL BMP4, 20 µM SB431542, and 20 µM SU5402 were each added, and these were subjected to culture for three weeks (Yabe, S. et al. Proc. Natl. Acad. Sci. U.S.A. 113, E2598-2607 (2016), Sudheer, S et al. Stem Cells Dev 21, 2987-3000 (2012)).

### (2) Human umbilical cord-derived high-potential pluripotent stem cells generate coenocytic cell according to differentiation induction methods [1] and [2] above.

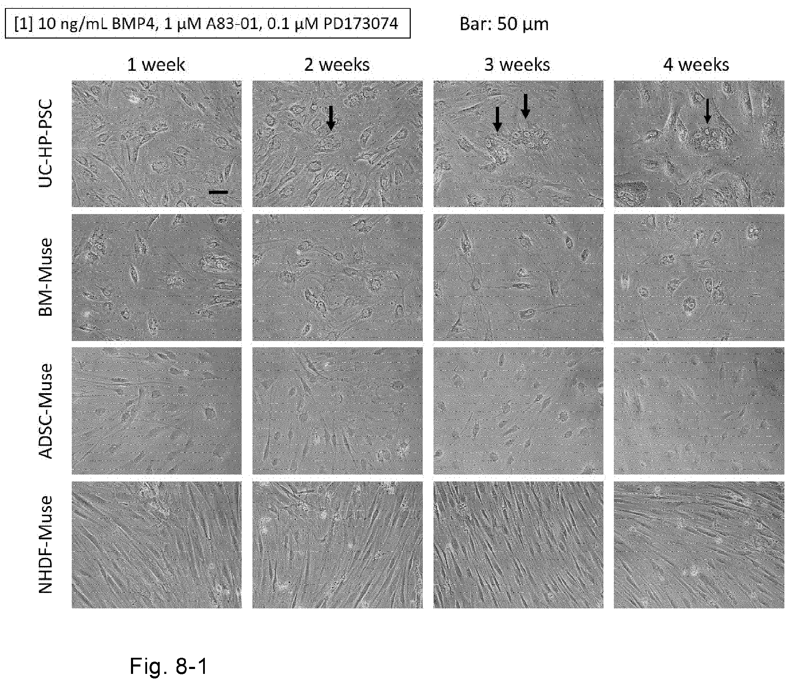

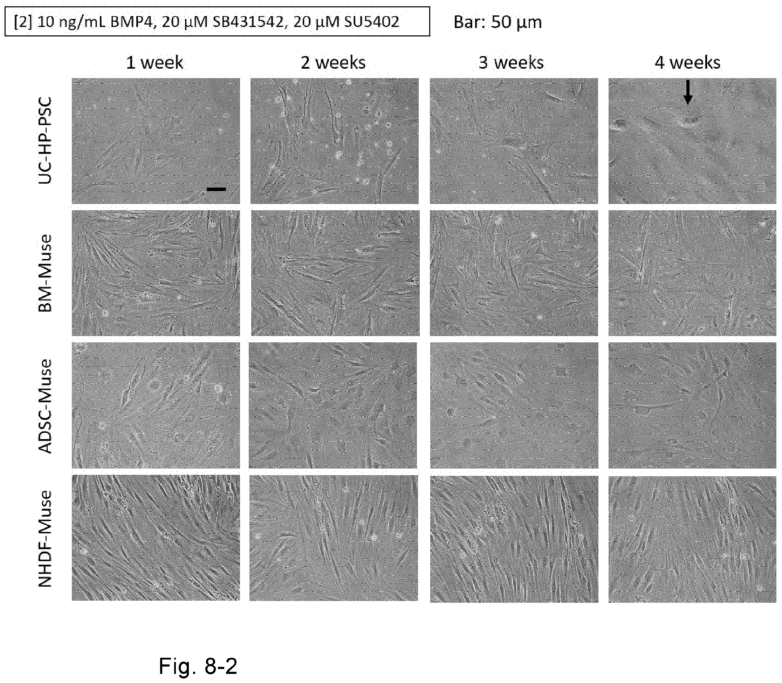

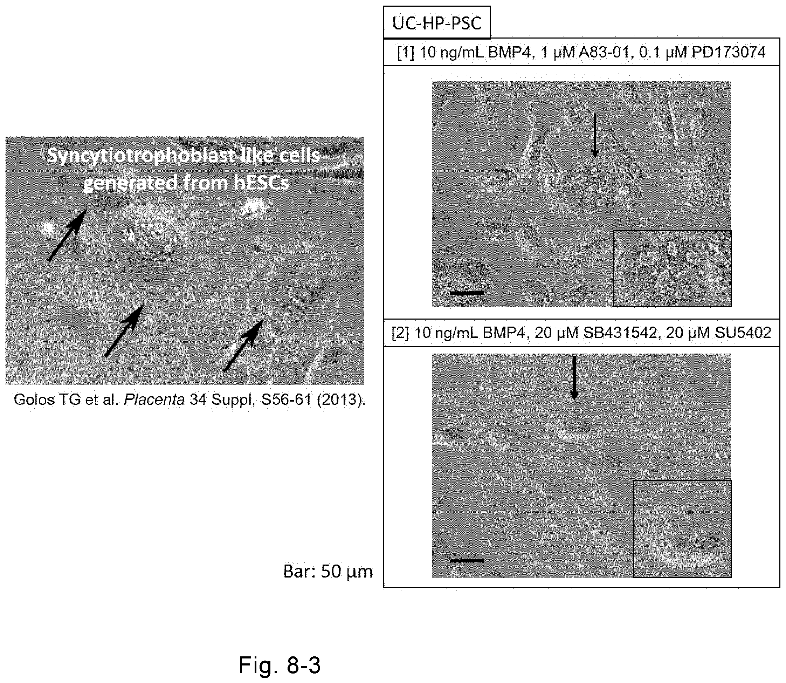

A placental syncytiotrophoblast-like cell is a coenocytic cell. As shown in FIG. 8, the human umbilical cord-derived SSEA-3-positive cells generated such a coenocytic cell on or after two weeks according to the induction method [1], and at three weeks according to the induction method [2]. The shape is very similar to the shape of a syncytiotrophoblast-like cells generated from a human ES cells. On the other hand, no coenocytic cell was observed in all the induction methods in the cases of bone marrow (BM)-, adipose (ADSC)-, and skin (NHDF)-derived Muse cells.

### (3) Expression of trophoblast marker in human umbilical cord-derived high-potential pluripotent stem cells, according to differentiation induction methods [1] and [2] above

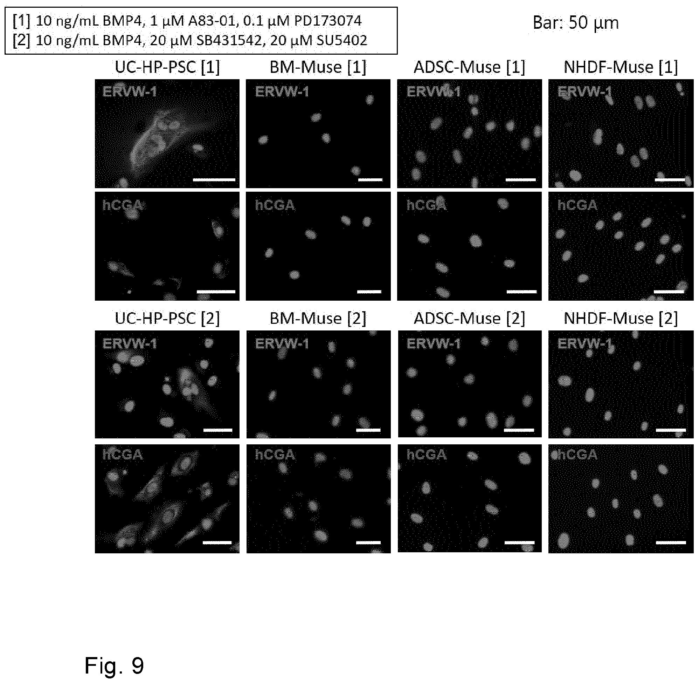

The cell was fixed by a 4% PFA/0.1 M phosphate buffer at three weeks after induction, and subjected to immunostaining on ERVW-1 and human Chorionic gonadotropin alpha chain (hCGA) as trophoblast markers. As a result, ERVW-1 and hCGA were detected in the human umbilical cord-derived SSEA-3-positive cells, but no expression in bone marrow-, adipose-, and skin-derived Muse cells was observed, in both the cases of induction methods [1] and [2], as shown in FIG. 9.

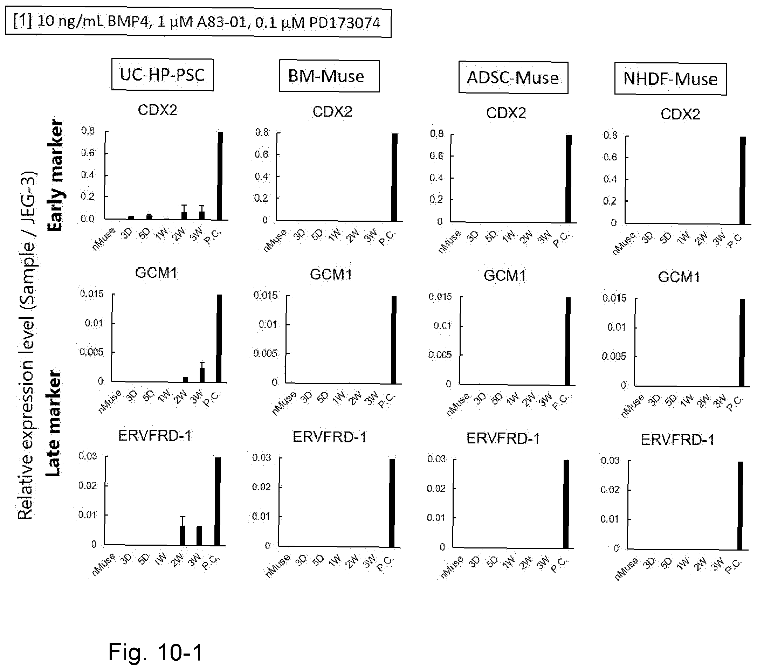

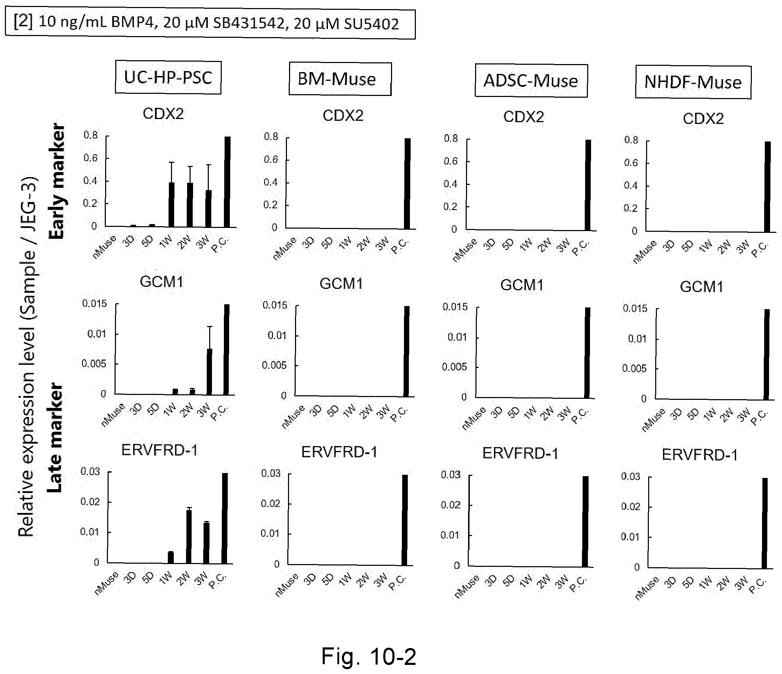

Expression of CDX2, GCM1, and ERVFRD-1 as trophoblast markers was evaluated on day 3 and day 5, and at one week, two weeks and three weeks after induction, by quantitative PCR. As a result, CDX2 as an early marker was detected in the human umbilical cord-derived S SEA-3-positive cells on or after three days from induction, and expression of GCM1 and ERVFRD-1 as late markers was observed on or after two weeks, as shown in FIGS. 10-1 and 10-2. It was considered that the induction method [2] allowed for higher expression of all the trophoblast markers and was a more efficient induction method than the induction method [1]. On the other hand, no expression of all the markers in bone marrow (BM)-, adipose (ADSC)-, and skin (NHDF)-derived Muse cells was observed.

It was thus indicated that the human umbilical cord-derived SSEA-3-positive cells had differentiation capacities into trophoblast lineages in extraembryonic tissue, unlike bone marrow-, adipose-, and skin-derived Muse cells.

### Example 1-5: CD133 as cell surface marker is positive in human umbilical cord tissue-derived high-potential pluripotent stem cells

CD133 is one of glycoproteins, and is a cell surface marker also known as Prominin 1. Although the function is still not clear, it has been revealed that such a marker is expressed in some stem/progenitor cells such as hematopoietic and neural stem cells.

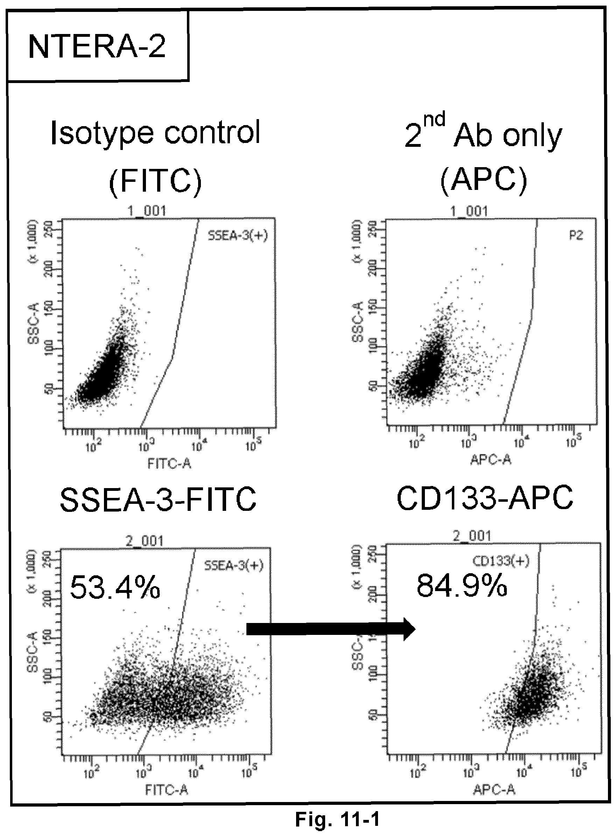

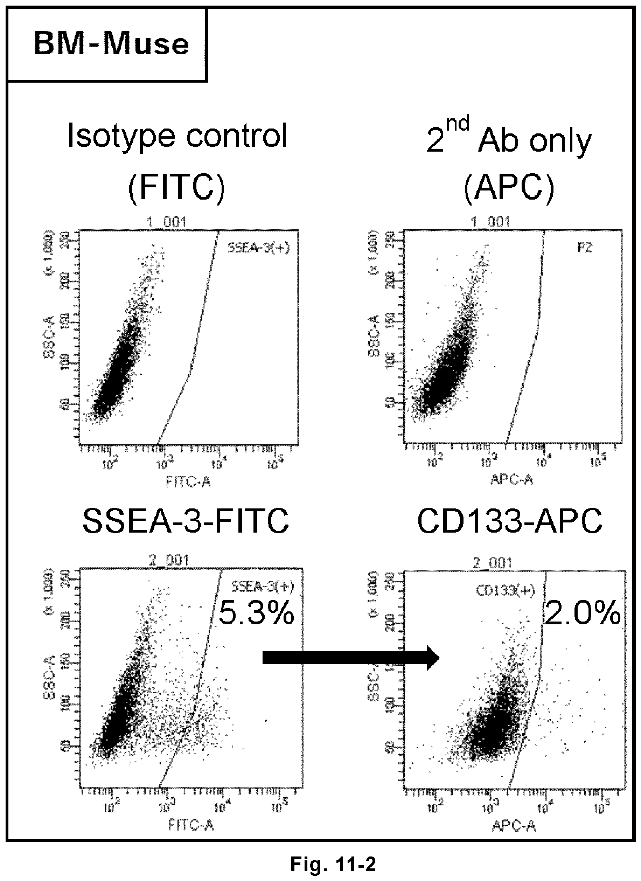

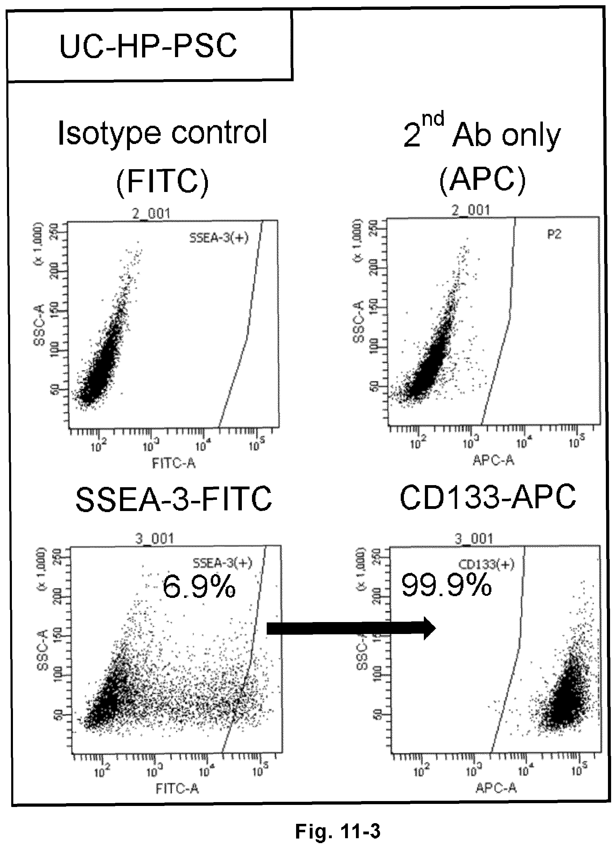

Expression of CD133 in the human umbilical cord-derived SSEA-3-positive cells obtained in Example 1-1 was confirmed by FACS. A bone marrow-derived Muse cell was used in a comparative group, and NTERA-2 as a pluripotent human embryonic cancer cell line was used as a positive control. As a result, as shown in FIGS. 11-1, 11-2 and 11-3, 84.9% of the SSEA-3-positive cells in NTERA-2 was CD133-positive, and 99.9% of the SSEA-3-positive cells in the human umbilical cord-derived S SEA-3-positive cells was CD133-positive. On the other hand, 2.0% of the SSEA-3-positive cells in the bone marrow-derived Muse cell was CD133-positive, and was a very low value as compared with that in the human umbilical cord-derived SSEA-3-positive cells.

### Example 1-6: Human umbilical cord tissue-derived high-potential pluripotent stem cells have differentiation capacities into germline cells

### (1) Induction of differentiation into primordial germ cells

Differentiation induction of the human umbilical cord-derived SSEA-3-positive cells obtained in Example 1-1 into primordial germ cells was confirmed by the following induction method, according to the following differentiation induction conditions for induction of differentiation of an iPS cells into primordial germ cells. A human bone marrow-derived Muse cells were used in a control group, a primordial germ cell (iPS-PGC) derived from an iPS cells was used in a positive control group. The human umbilical cord-derived SSEA-3-positive cells or human bone marrow-derived Muse cells were seeded at a density of 5 × 10⁴ cells/cm² on a dish, DMEM-low-glucose, 10% FBS, 50 ng/mL Activin A, 3 µM CHIR99021, and 10 µM Y-27632 were added, and these were subjected to adherent culture for two days. Thereafter, the cell was seeded with a Low-cell binding V-bottom 96-well plate so as to be at 3 × 10³ cells/well, DMEM-low-glucose, 10% FBS, 200 ng/mL BMP-4, 1000 U/mL LIF, 100 ng/mL SCF, 50 ng/mL EGF, and 10 µM Y-27632 were added, and these were subjected to suspension culture for 4 days.

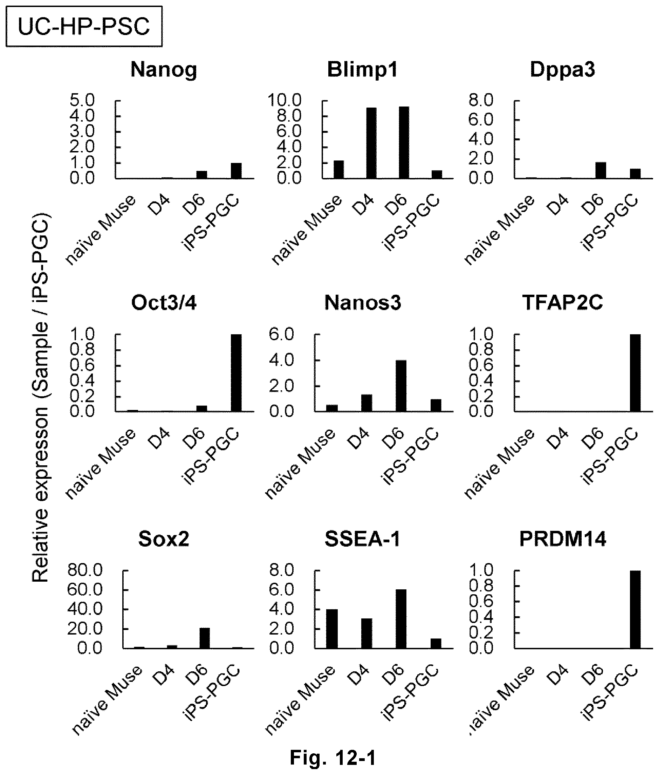

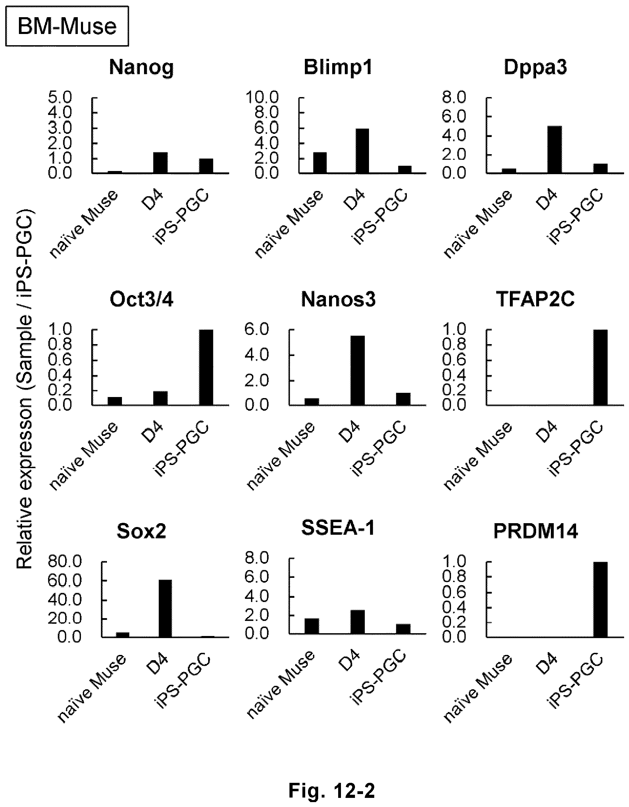

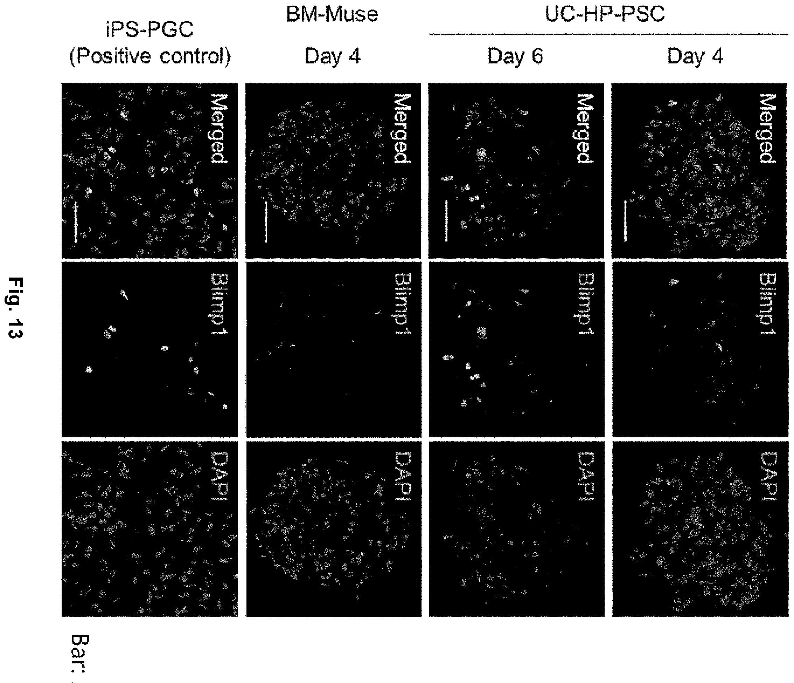

Expression of Blimp1, Nanos3, Dppa3, TFAP2C, PRDM14, and SSEA-1 as germline markers on day 4 and day 6 after induction was evaluated by quantitative PCR. The results are shown in FIGS. 12-1 and 12-2. Blimp1 and Nanos3 were detected in the human umbilical cord-derived SSEA-3-positive cells on or after four days from induction. Expression of Dppa3 was observed and expression of SSEA-1 was also enhanced at six days from induction. On the other hand, expression of some markers such as Nanos3 and Dppa3 in the bone marrow-derived Muse cells were observed, but expression of Blimp1 and SSEA-1 exhibited low values as compared with those in the human umbilical cord-derived SSEA-3-positive cells. These cells were fixed by a 4% PFA/0.1 M phosphate buffer on day 4 and day 6 after induction, and subjected to immunostaining on Blimp1 as a germline marker. The results are shown in FIG. 13. Blimp 1 was detected in the human umbilical cord-derived SSEA-3-positive cells, but almost no expression thereof was observed in the bone marrow-derived Muse cell. It was thus found that the human umbilical cord-derived SSEA-3-positive cells had more efficient differentiation capacities into germline cells than the bone marrow-derived Muse cells.

### (2) Human umbilical cord-derived high-potential pluripotent stem cells more efficiently differentiate into germline cells due to phagocytosis of primordial germ cell induced to cell death

A commercially available human umbilical cord-derived SSEA-3-positive cells were used to confirm induction of differentiation into primordial germ cells by the following induction method. The human umbilical cord-derived SSEA-3-positive cells were seeded at a density of 5 × 10³ cells/cm² on a dish, thereafter male or female mouse primordial germ cells induced to cell death by 100 µM Rotenone and 100 µM Oligomycin A were added at a density of 1 × 10⁴ cells/cm² and subjected to phagocytosis in DMEM-low-glucose and 10% FBS for three days. Thereafter, the following three types of induction were tried. <1> After the mouse primordial germ cells induced to cell death were removed, the cells were subjected to adherent culture in DMEM-low-glucose and 10% FBS for 11 days. <2> The human umbilical cord-derived SSEA-3-positive cells were seeded at a density of 5 × 10³ cells/cm² on a dish, thereafter any somatic cell in the genital ridge, other than a primordial germ cells subjected to mitomycin C treatment, were added so as to be at a density of 5 × 10³ cells/cm², and these were subjected to adherent culture in DMEM-low-glucose and 10% FBS for 11 days. <3> The human umbilical cord-derived SSEA-3-positive cells and any somatic cell in the genital ridge, other than the primordial germ cells subjected to mitomycin C treatment, were suspended with a Low-cell binding V-bottom 96-well plate so as to be respectively at 5 × 10³ cells/well and 5 × 10⁴cells/well, and these were subjected to suspension culture in DMEM-low-glucose and 10% FBS for 11 days.

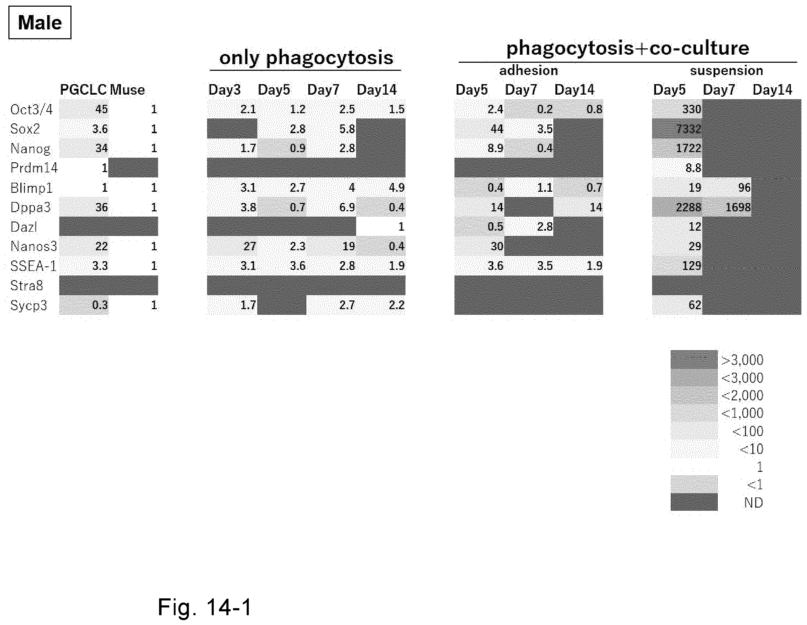

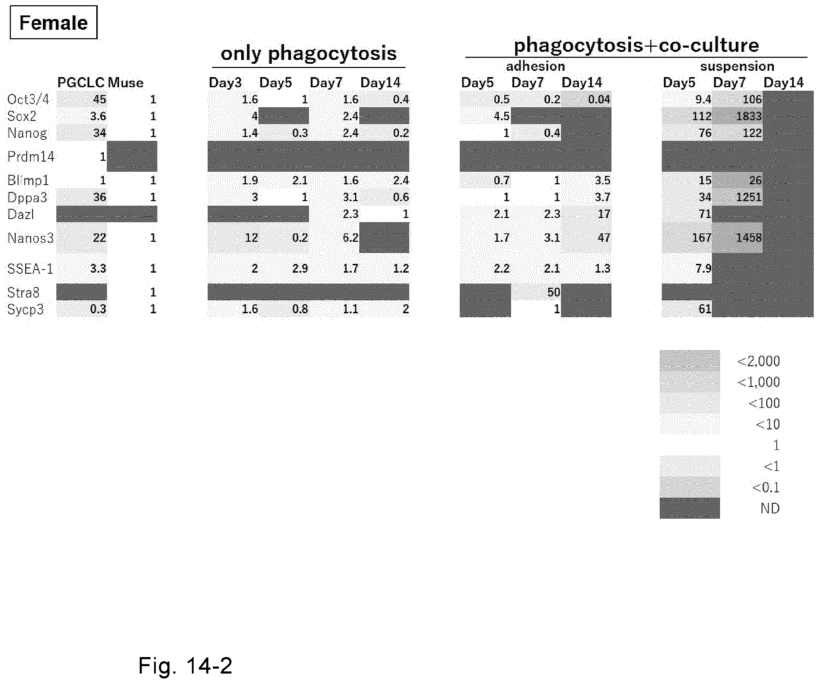

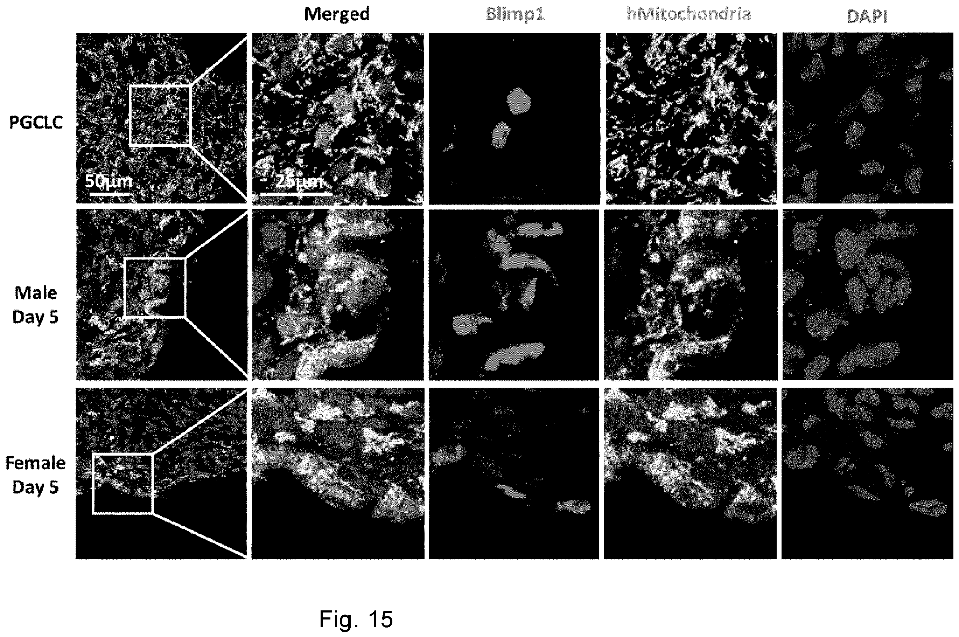

Expression of PRDM14, Blimp 1, Dppa3, Dazl, Nanos3, SSEA-1, Stra8, and Sycp3 as germline markers on day 3, day 5, day 7, and day 14 after induction was evaluated by quantitative PCR. A primordial germ cell-like cell (PGCLC) derived from a human iPS cells was used in a positive control group. The results are shown in FIGS. 14-1 and 14-2. It was observed in the induction method <1> that expression of markers such as Blimp1, Dppa3, and Nanos3 was increased even in human umbilical cord-derived SSEA-3-positive cells phagocytosed each of the female and male primordial germ cells. It was observed in the induction method <2> that expression of some of the markers exhibited higher values, but expression of most of the markers was comparable with in the induction method <1>. It was observed in the induction method <3> that expression of all the markers exhibited high values on day 5 after induction, and the induction method was indicated to be a more efficient induction method of differentiation into germline cells than such other induction methods. However, the number of cells died due to the influence of suspension culture was increased on or after seven days, thereby leading to deterioration in expression. The cells were then fixed by a 4% PFA/0.1 M phosphate buffer on day 5 in the induction method <3> and immunostaining on human mitochondria and Blimp1 as a germline marker was performed. The results are shown in FIG. 15. Expression of Blimp 1 in a human mitochondria-positive cells was confirmed in human umbilical cord-derived SSEA-3-positive cells phagocytosed each of the female and male primordial germ cells. It was thus found that the human umbilical cord-derived SSEA-3-positive cells efficiently differentiated into germline cells due to phagocytosis of primordial germ cell induced to cell death.

The commercially available human umbilical cord-derived SSEA-3-positive cells used in the present Examples was a human umbilical cord-derived high-potential pluripotent stem cell fraction including 91.2% of CD133-positive cells.

The human umbilical cord-derived SSEA-3-positive cells obtained in Example 1 have useful characteristics of pluripotent stem cells as in conventional Muse cells obtained from the bone marrow, peripheral blood, fat, skin, and/or the like, additionally has differentiation capacities into any extraembryonic tissue cells and/or germline cells and have differentiation capacities close to totipotency, and furthermore has characteristics, for example, positivity of CD133 as a cell surface marker, according to other analysis in Example 1. Thus, a CD133-positive pluripotent stem cells having differentiation capacities into any extraembryonic tissue cells and/or germline cells, exemplified by the human umbilical cord-derived SSEA-3-positive cells obtained in Example 1, is defined as a novel SSEA-3-positivepluripotent stem cells and are referred to as "high-potential pluripotent stem cells" of the present invention, and in particular, human umbilical cord-derived one is referred to as "human umbilical cord-derived high-potential pluripotent stem cells."

### Example 2-1: Isolation of human amnion-derived high-potential pluripotent stem cells

A high-potential pluripotent stem cells were obtained from human amnion according to the method for isolation and identification of human Muse cells described in WO2011/007900.

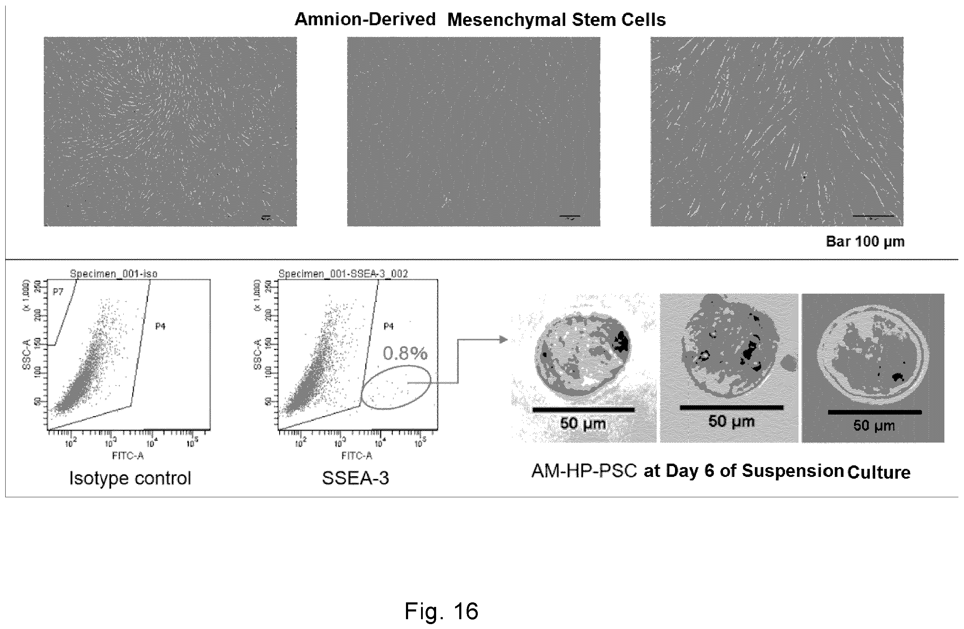

After the resulting human amnion-derived mesenchymal stem cells were expansively/continuously cultured, a human amnion-derived SSEA-3-positive cells (namely, human amnion-derived high-potential pluripotent stem cells: AM-HP-PSC) were isolated with an anti-SSEA-3 antibody. As a result, about 1% of the SSEA-3-positive cells was present in the human amnion-derived mesenchymal stem cells, as shown in FIG. 16. In addition, single cell suspension culture of the resulting human amnion-derived SSEA-3-positive cells was performed, and thus about 25% of the SSEA-3-positive cells formed an embryoid body-like cluster on day 6 of the single cell suspension culture (FIG. 16).

### Example 2-2: Study of characteristics of human amnion-derived high-potential pluripotent stem cells

### (1) Clusters formed from human amnion-derived high-potential pluripotent stem cells in adherent culture and differentiation into triploblastic-lineage cells

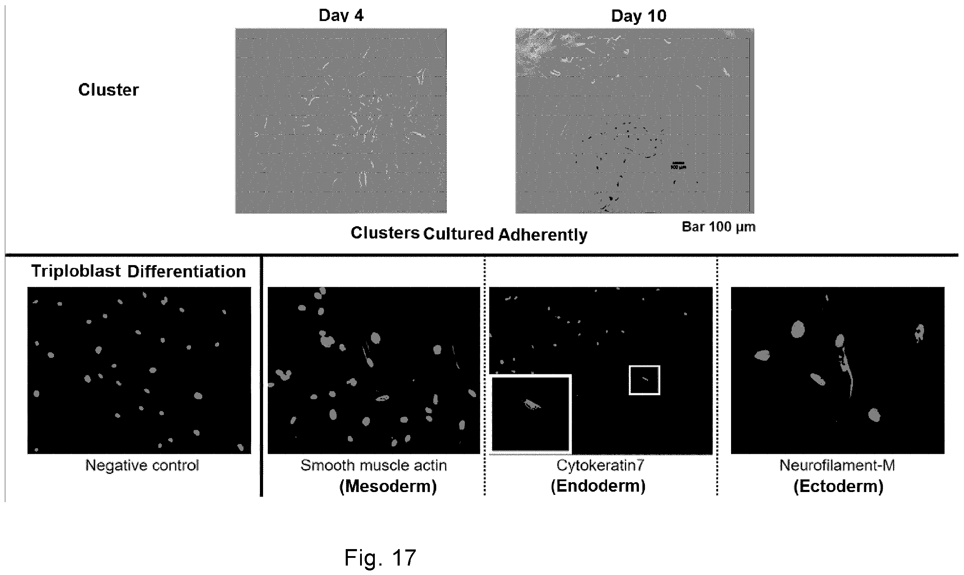

In order to confirm differentiation capacities into triploblastic-lineage cells, a pluripotent cluster of a single human amnion-derived SSEA-3-positive cell was transferred to a dish coated with gelatin, and subjected to adherent culture. After about 10 days, cytokeratin7 (CK7, endoderm marker), α-smooth muscle actin (SMA, mesoderm marker), and neurofilament-M (ectoderm marker) were detected by immunostaining (FIG. 17). It was found from the results that the human amnion-derived SSEA-3-positive cells had spontaneous differentiation capacities into triploblastic-lineage cells.

### (2) Analysis of cell surface markers of human amnion mesenchymal stem cell (MSC) and human amnion-derived high-potential pluripotent stem cells

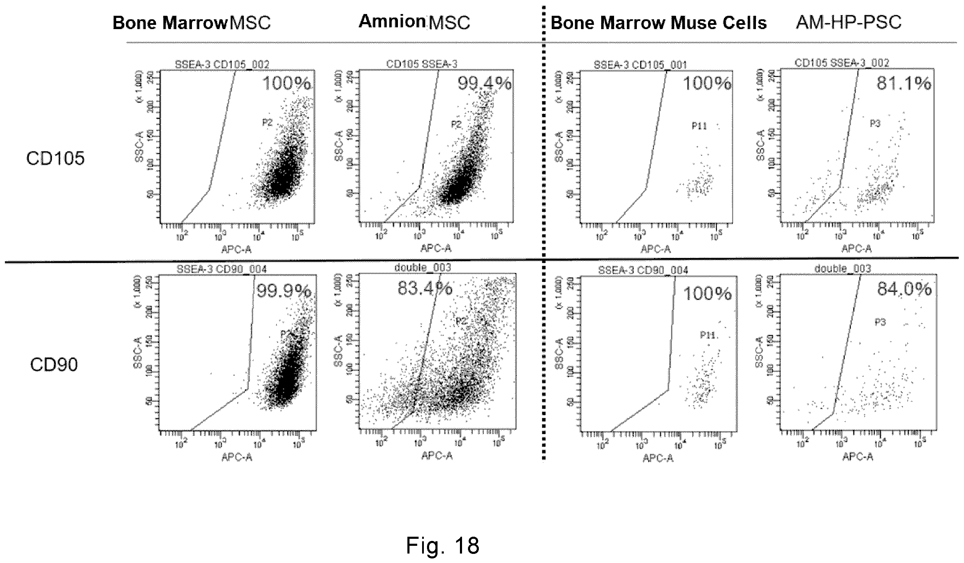

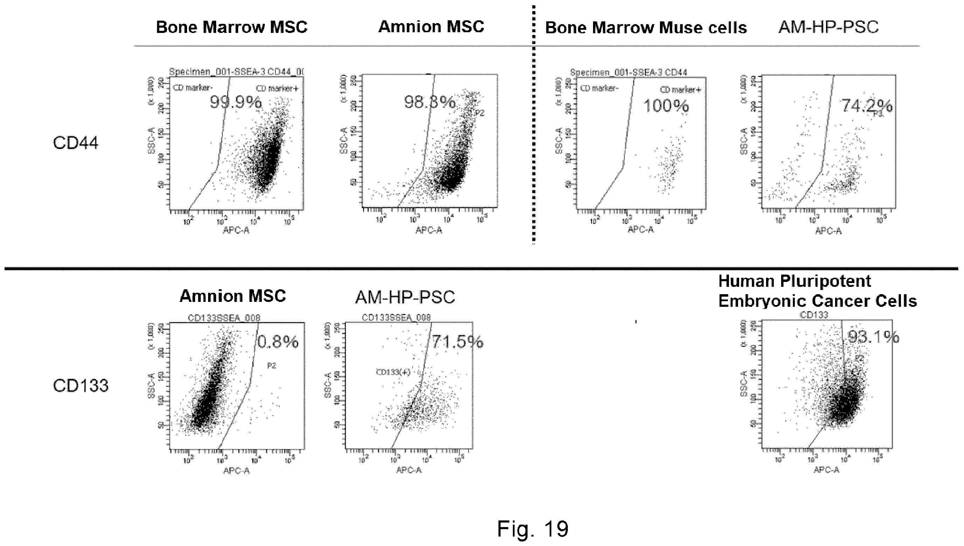

In the human amnion-derived SSEA-3-positive cells, the double positivity of SSEA-3 and CD105 was 81.1%, the double positivity of SSEA-3 and CD90 was 84.0%, and the double positivity of SSEA-3 and CD44 was 74.2%. In the human amnion-derived SSEA-3-positive cells, the double positivity of SSEA-3 and CD133 was 71.5% (FIGS. 18 and 19).

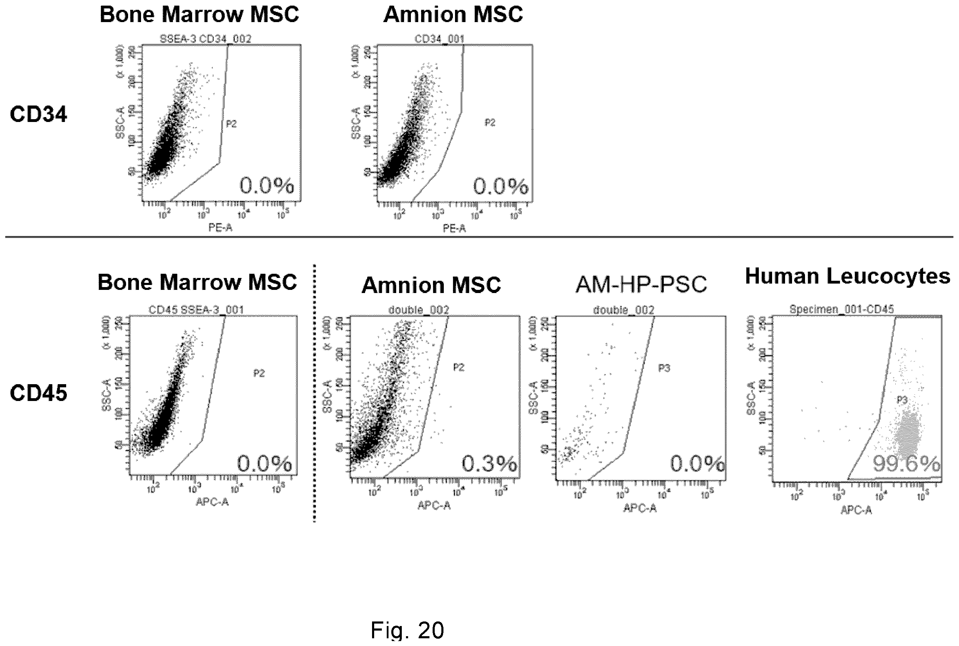

Hematopoietic cell surface markers such as CD34 and CD45 were almost not expressed in the human amnion-derived MSCs and human amnion-derived SSEA-3-positive cells, as in the bone marrow-derived MSCs and Muse cells (FIG. 20).

### (3) Expression analysis of genes of human amnion-derived high-potential pluripotent stem cells and conventional human bone marrow-derived Muse cells

### 1) Expression analysis of genes (Blimp1/Dppa3/ITGA/Sycp3/TFAP2c/TP63) specific for germline cells

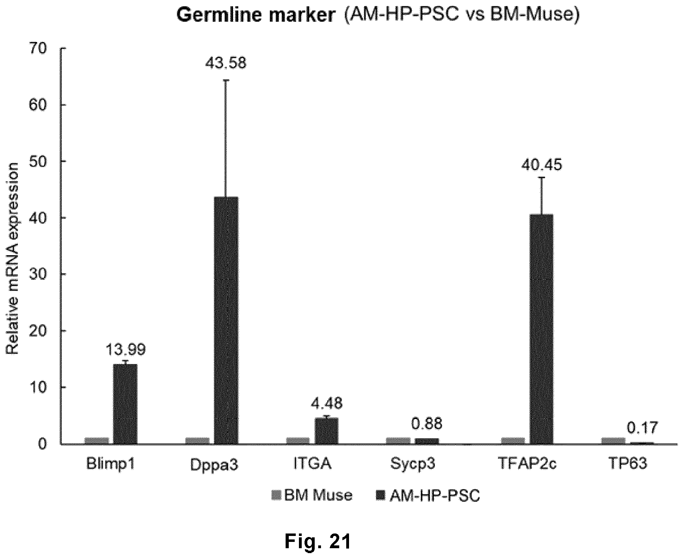

Gene expressions specific for germline cells of the human amnion-derived SSEA-3-positive cells and human bone marrow-derived Muse cells were comparatively analyzed by quantitative PCR. As a result, expression of Blimp 1, Dppa3, ITGA, and TFAP2c as germline markers in the human amnion-derived SSEA-3-positive cells was very high as compared with those in the bone marrow-derived Muse cells, as shown in FIG. 21. These results indicated capacities of differentiation of the human amnion-derived SSEA-3-positive cells into germline cells, and furthermore indicated capacities of induction of differentiation into extraembryonic tissue cells through differentiation into triploblastic-lineage cells.

### 2) Gene indicating pluripotency

### (a) Primed pluripotency marker (Oct3/4/Nanog/Sox2)

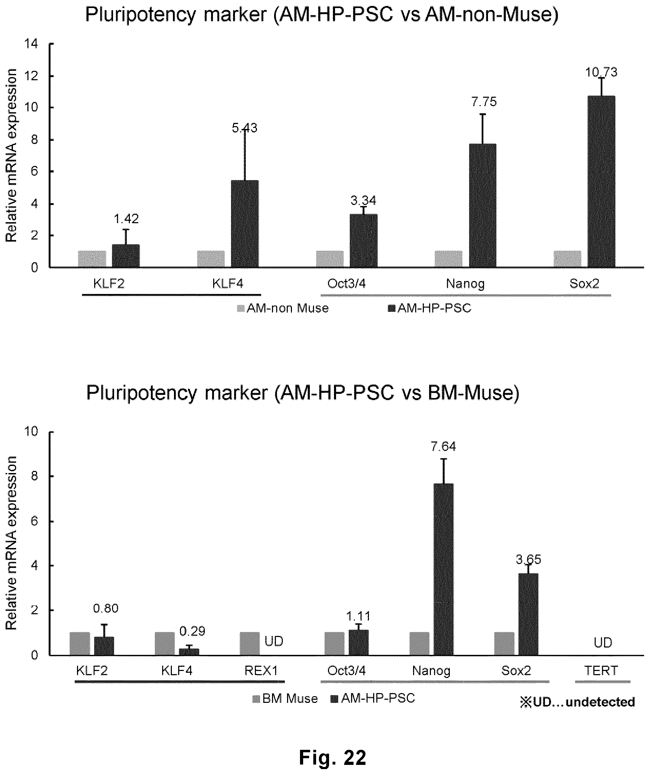

Expression of Oct3/4, Nanog, and Sox2 as Primed pluripotency markers in the human amnion-derived SSEA-3-positive cells, human amnion-derived SSEA-3 negative cells (AM-non-Muse) and human bone marrow-derived Muse cells were comparatively analyzed by quantitative PCR. High expression of the primed pluripotency markers were observed in the human amnion-derived SSEA-3-positive cells, as compared with in the bone marrow-derived Muse cells (FIG. 22). These results indicated that the human amnion-derived SSEA-3-positive cells exhibited high pluripotency as compared with the bone marrow-derived Muse cells and human amnion-derived SSEA-3 negative cells.

### (b) Naïve pluripotency marker (KLF2/KLF4/REX1)

Expression of KLF2, KLF4, and REX1 as naive pluripotency markers in the human amnion-derived SSEA-3-positive cells, human amnion-derived SSEA-3 negative cells (AM-non-Muse) and human bone marrow-derived Muse cells was comparatively analyzed by quantitative PCR. No difference in expression was observed between the human amnion-derived SSEA-3-positive cells and bone marrow Muse cells (FIG. 22). On the other hand, high expression was observed in the human amnion-derived SSEA-3-positive cells, as compared with the human amnion-derived SSEA-3 negative cells.

### (c) Telomerase reverse transcriptase gene (TERT)

Expression of telomerase reverse transcriptase genes (TERT) in the human amnion-derived SSEA-3-positive cells and human bone marrow-derived Muse cells were comparatively analyzed by quantitative PCR. No expression was observed in both the human amnion-derived SSEA-3-positive cells and the bone marrow Muse cells (FIG. 22). These characteristics were suggested to be characteristics (non-tumorigenesis) common in SSEA-3-positive pluripotent stem cells, regardless of any original cell tissue.

The human amnion-derived SSEA-3-positive cells obtained in Example 2 have useful characteristics of pluripotent stem cells as in conventional Muse cells obtained from bone marrow, peripheral blood, fat, skin, and/or the like, additionally have differentiation capacities into any extraembryonic tissue cells and/or germline cells and have differentiation capacities close to totipotency, and furthermore has characteristics, for example, positivity of CD133 as a cell surface marker, at about 70% or more. Thus, the presence of the "high-potential pluripotent stem cells" of the present invention, also in human amnion as one extraembryonic tissue, has been revealed in Example 2. In particular, the human amnion-derived SSEA-3-positive cells obtained in Example 2 can be referred to as "human amnion-derived high-potential pluripotent stem cell fraction" including of 70% or more of human amnion-derived SSEA-3 and CD133 double-positive high-potential pluripotent stem cells.

### Example 3-1: Isolation of human placenta tissue-derived high-potential pluripotent stem cells

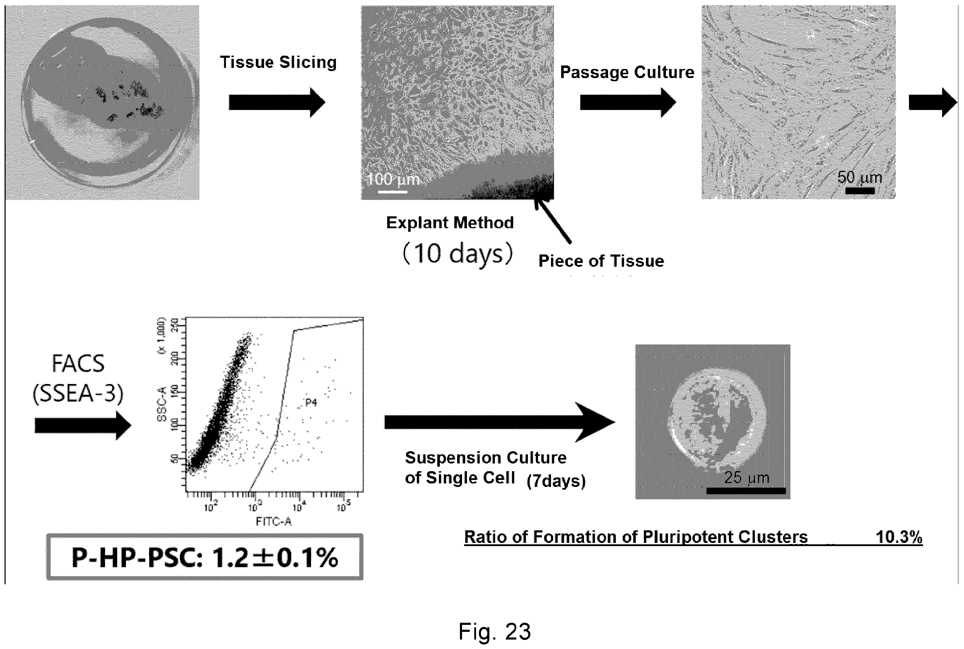

High-potential pluripotent stem cells can be obtained from human placenta tissue according to the method for isolation and identification of human Muse cells described in WO2011/007900. More specifically, a human placenta tissue-derived high-potential pluripotent stem cells were obtained by FACS isolation with SSEA-3 as an index, according to a process in FIG. 23. Specifically, human placenta was sliced to a size of 0.5-mm square, and each tissue section was placed on a 10-cm dish at a 1-cm interval. αMEM, 15% FBS, 1 ng/mL bFGF, and 2 mM GlutaMAX were added, and the resultant was subjected to culture for about 10 days according to an explant method. After established human placenta tissue-derived mesenchymal stem cells were expansively/continuously cultured, a human placenta tissue-derived SSEA-3-positive cells were isolated with an anti-SSEA-3 antibody. About 1% of the SSEA-3-positive cells was present in the established human placenta-derived mesenchymal stem cells. The human placenta-derived SSEA-3-positive cells isolated by FACS was subjected to single cell suspension culture, and 10.3% of the SSEA-3-positive cells formed embryoid body-like clusters on day 7 of the culture.

### Example 3-2: CD133 as cell surface marker is positive in human placenta tissue-derived high-potential pluripotent stem cells

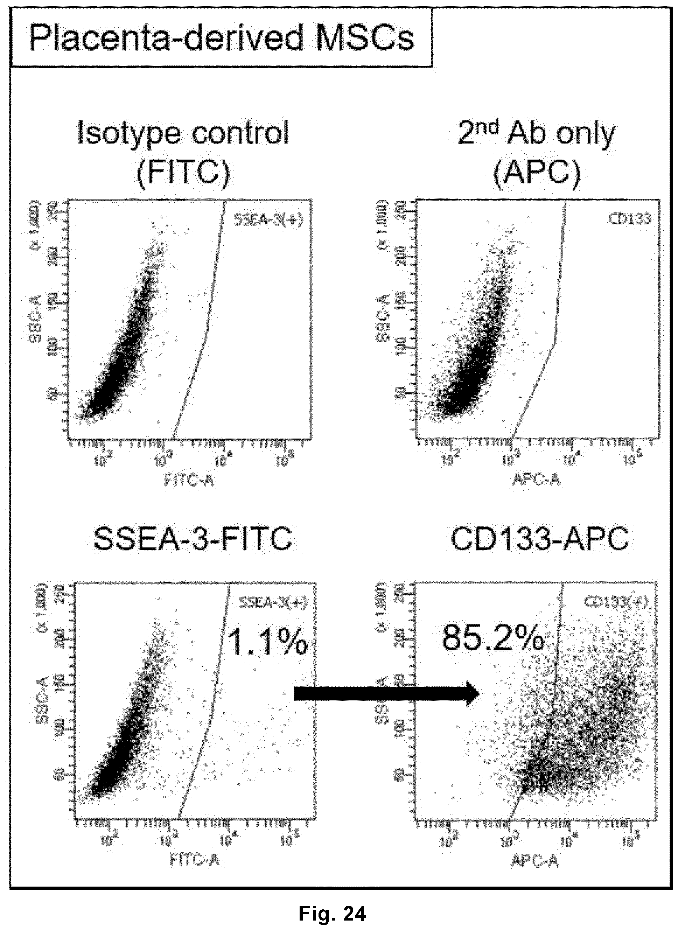

Expression of CD133 in the human placenta-derived SSEA-3-positive cells obtained in Example 3-1 was confirmed by FACS. As a result, 85.2% of the human placenta-derived S SEA-3-positive cells was CD133-positive, as shown in FIG. 24.

Therefore, the human placenta tissue-derived SSEA-3-positive cells obtained in Example 3 can be referred to as "human placenta-derived high-potential pluripotent stem cell fraction" including 80% or more of a human placenta-derived high-potential pluripotent stem cells (P-HP-PSC).

Example 3-3: Human placenta-derived high-potential pluripotent stem cells more efficiently differentiated into germline cells due to phagocytosis of primordial germ cell induced to cell death

The human placenta-derived high-potential pluripotent stem cell fraction obtained in Example 3-1 was used to confirm induction of differentiation into primordial germ cells by the following induction method. The SSEA-3-positive cells of the human placenta-derived high-potential pluripotent stem cell fraction was seeded at a density of 5 × 10³ cells/cm² on a dish, thereafter a male or female mouse primordial germ cells induced to cell death by 100 µM Rotenone and 100 µM Oligomycin A were added at a density of 1 × 10⁴ cells/cm², and subjected to phagocytosis in DMEM-low-glucose and 10% FBS for three days. After the mouse primordial germ cells induced to cell death were removed, the cells were subjected to adherent culture in DMEM-low-glucose and 10% FBS, 20 µM 5-Azacytidine, and 10 µM Retinoic acid for three days, and then cell suspension with a Low-cell binding V-bottom 96-well plate so that a density of 5 × 10³ cells/well was obtained, and suspension culture was performed in DMEM-low-glucose, 10% FBS for two days.

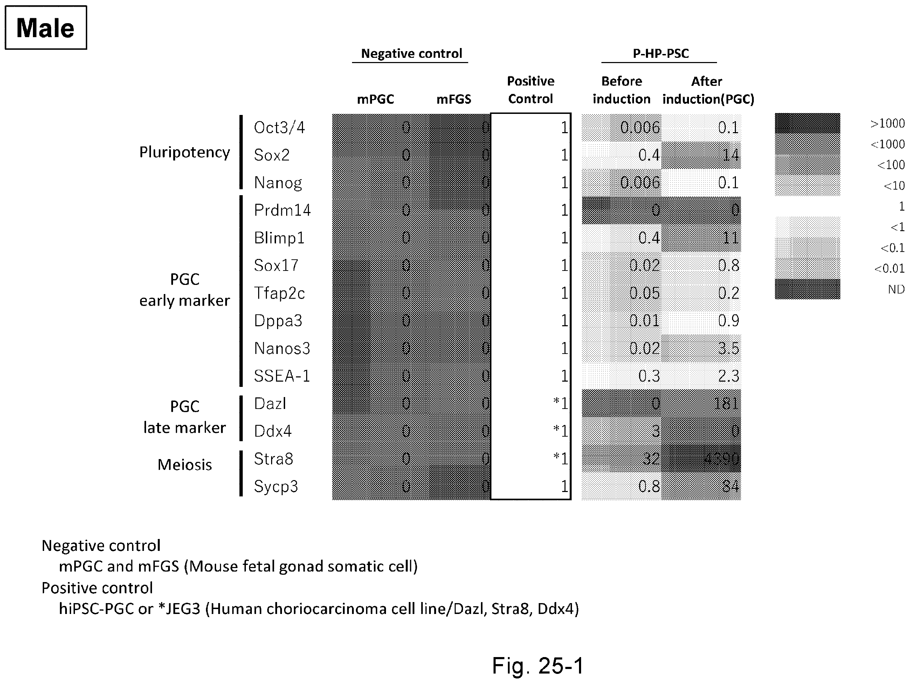

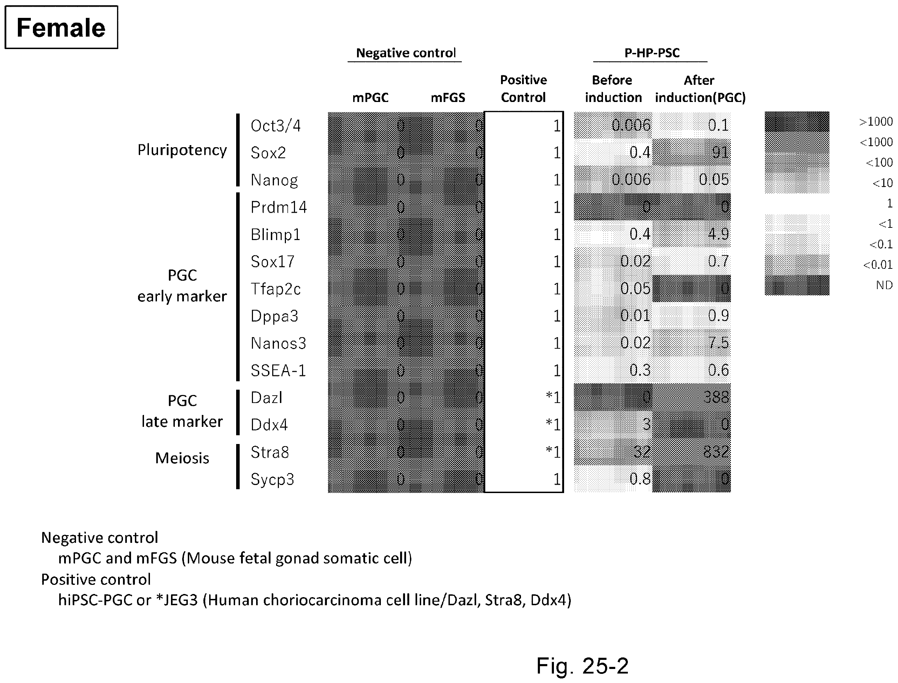

Expression of PRDM14, Blimp1, Sox17, Tfap2c, Dppa3, Nanos3, and SSEA-1 as early markers of germline cells, Dazl and Ddx4 as late markers, and Stra8 and Sycp3 as meiotic markers, on day 8 after induction, was evaluated by quantitative PCR. A primordial germ cell-like cells (PGCLC) induced from a human iPS cells or a human gestational choriocarcinoma cell line JEG3 were used in a positive control group. The results are shown in FIGS. 25-1 and 25-2. Increases in early markers such as Blimp1 and Nanos3 were observed in human umbilical cord-derived high-potential pluripotent stem cells phagocytosed each of the female and male primordial germ cells, and expression of, in particular, Dazl as a late marker and Stra8 as a meiotic marker was remarkably increased. It was thus found that the human placenta-derived high-potential pluripotent stem cell fraction efficiently differentiated into germline cells due to phagocytosis of primordial germ cells induced to cell death, as in the human umbilical cord-derived high-potential pluripotent stem cell fraction.

Therefore, the high-potential pluripotent stem cells of the present invention are suggested to have differentiation capacities into germline cells and have differentiation capacities close to totipotency.

Example 4: Human umbilical cord tissue and placenta-derived high-potential pluripotent stem cells allow for higher expression of HLA-G than that of bone marrow-, adipose-, and skin-derived Muse cells

HLA-G is expressed in an extravillous trophoblast constituting the outermost layer of placenta where fetal tissue invades maternal tissue, and has an immunosuppression function for allowing the fetus to escape from the immune mechanism of the mother.

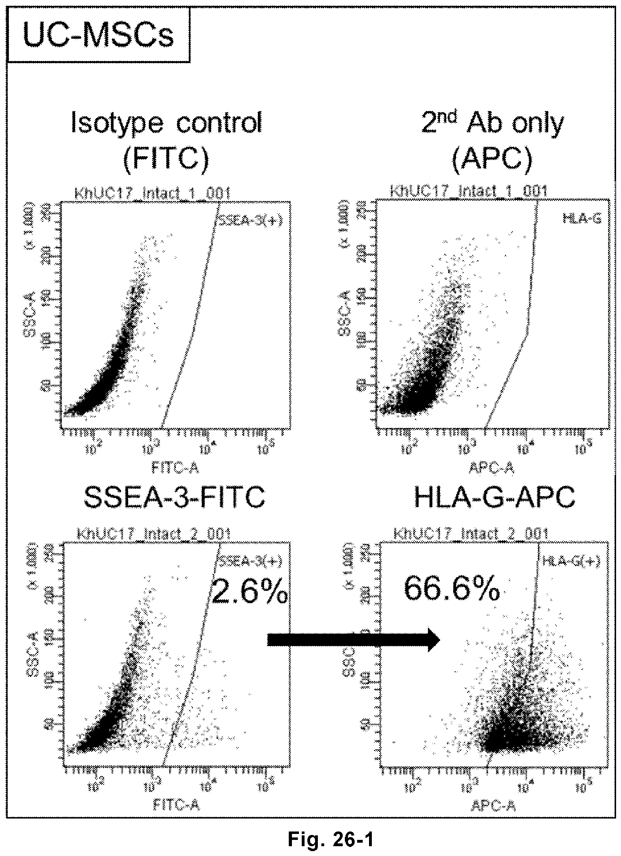

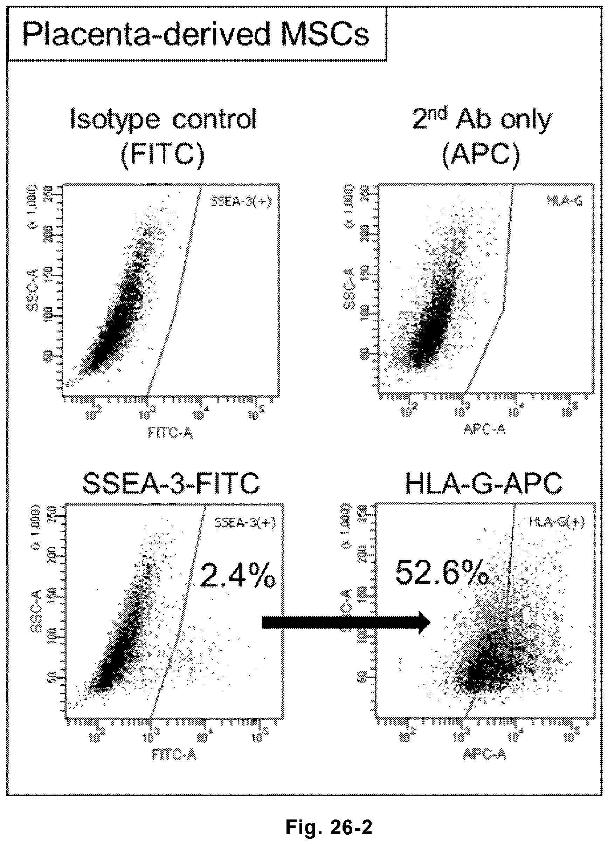

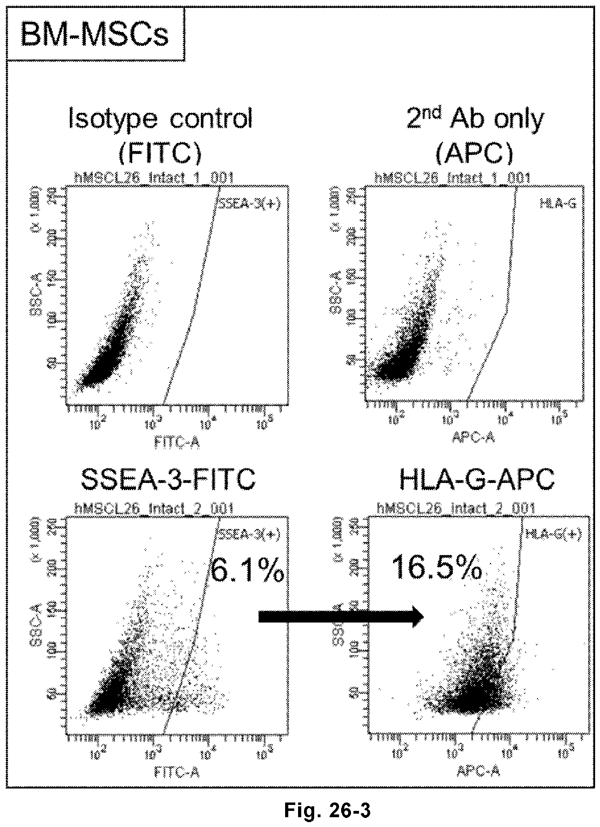

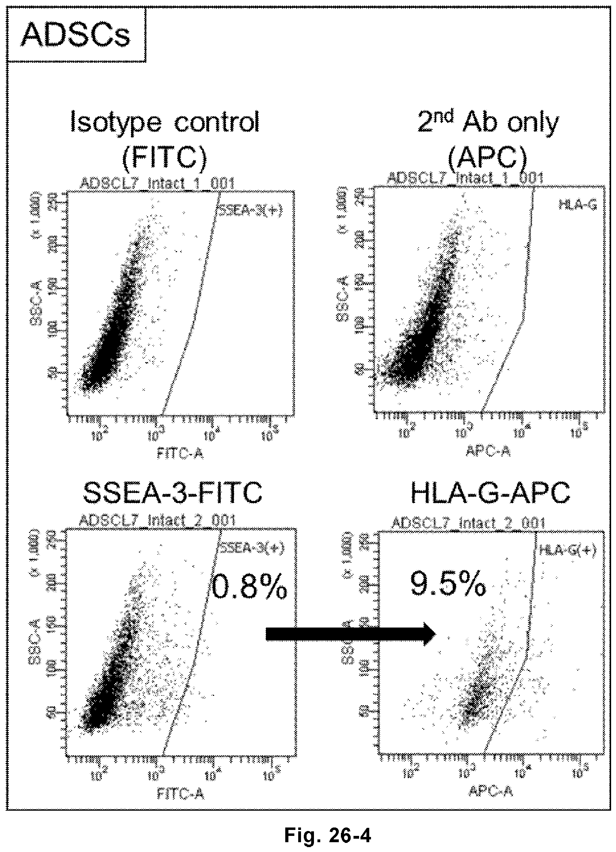

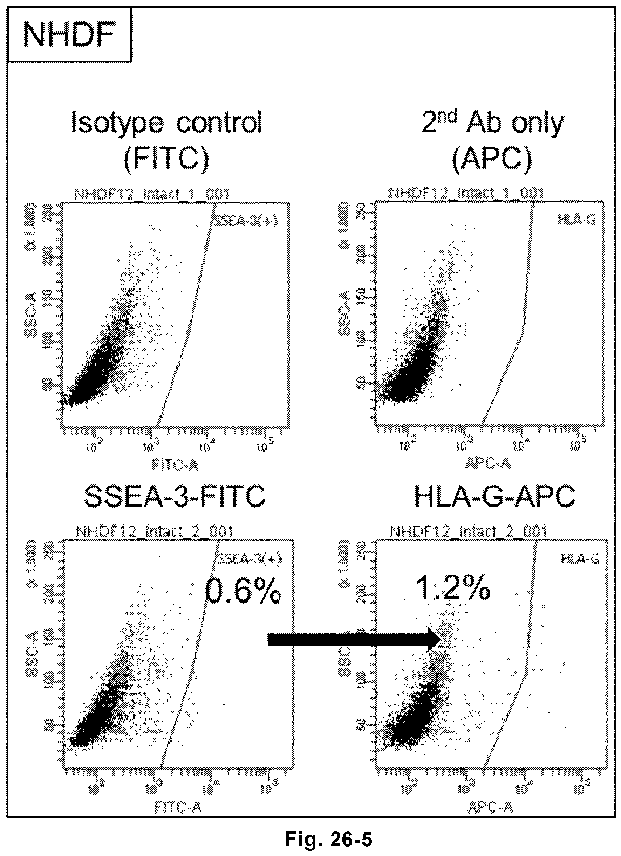

Expression of HLA-G was confirmed by FACS, with the human umbilical cord-derived high-potential pluripotent stem cell fraction obtained in Example 1-1 and the human placenta-derived high-potential pluripotent stem cell fraction obtained in Example 3-1. Bone marrow-, adipose-, and skin-derived Muse cells were used in comparative groups. As a result, as shown in FIGS. 26-1, 26-2, 26-3, 26-4 and 26-5, and Table 2, the respective proportions of the HLA-G-positive cells in the SSEA-3-positive cells in human umbilical cord (UC)- and human placenta (Placenta)-derived high-potential pluripotent stem cell fractions were 66.6% and 52.6%. On the other hand, the respective proportions of the HLA-G-positive cells in the SSEA-3-positive cells in the bone marrow (BM)-, adipose (ADSC)-, and skin (NHDF)-derived Muse cells were 16.5%, 9.5%, and 1.2%, and were very low values as compared with those in the human umbilical cord and human placenta-derived high-potential pluripotent stem cell fractions.

Therefore, the high-potential pluripotent stem cells of the present invention allow for high expression of HLA-G, and is suggested to also have a high immunomodulatory function as compared with Muse cells and is suggested to have usability such as a reduction in risk of rejection in donor preparation administration.

**Table 2**

| | HLA-G (%) in SSEA-3-positive cell |
|---|---|
| UC-HP-PSC | 66.6% |
| P-HP-PSC | 52.6% |
| BM-Muse | 16.5% |
| ADSC-Muse | 9.5% |
| NHDF-Muse | 1.2% |

### Example 5: Effect of human umbilical cord-derived high-potential pluripotent stem cells in rat myocardial infarction model

### (1) Production of rat myocardial infarction model

The protocol about the experimental animals used in the present study was carried out with the council/approval obtained from the animal testing committee in Shiga Laboratory of Nissei Bilis Co., Ltd. The surgery of myocardial infarction model production was performed on the day before specimen administration. Specifically, each rat was anesthetized by subcutaneous administration of three types of mixed anesthetic agents at a volume of 2.5 mL/kg, and fixed at the supine position, a tracheal tube was inserted through the mouth into the respiratory tract, artificial respiration was applied by an artificial respirator for small animals, thoracotomy at the left chest sidewall was applied to expose the heart, and thereafter the left anterior descending coronary artery (LAD) was occluded with a suture needle (ELP suture needle M10-50B2, Akiyama-seisakusyo. Co., Ltd.) for 30 minutes. An electrocardiogram (Lead II) was here measured with LabChart-Pro (AD Instruments), and an increase in ST electric potential and whitening of the cardiac muscle were macroscopically observed, to confirm the presence of occlusion. If ventricular fibrillation (VF) occurred, resuscitation treatment was performed. A myocardial infarction model (ischemia/reperfusion) was produced by reperfusion after 30 minutes of occlusion.

After reperfusion, the chest was closed, and the incision sites was sutured. Here, a proper amount of a liquid having a concentration of 2 mg/mL of ampicillin sodium for injection (VICCILLIN FOR INJECTION, 0.5 g, Meiji Seika Pharma Co., Ltd.) for infection prevention was dropped onto the incision sites. After suturing, sterilization was made with a povidone iodine preparation (Popiyodon Solution 10%, Yoshida Pharmaceutical Company Limited). Thereafter, atipamezole (Atipane Injection, Kyoritsu Seiyaku Corporation) at a dose of 0.15 mg/kg was subcutaneously administrated, followed by returning to a case after awakening. The day following the myocardial infarction model production was defined as day 0. An analgesic Meloxicam (Metacam 0.5% injection, Boehringer Ingelheim Animal Health Japan Co., Ltd.) was subcutaneously administrated for two days from the day following the surgery of myocardial infarction model production.

### (2) Evaluation (echocardiographic measurement) in rat myocardial infarction model

The human bone marrow-derived Muse cells (BM-Muse), the human umbilical cord-derived high-potential pluripotent stem cell fraction (UC-HP-PSC) obtained in Example 1-1 and the human bone marrow-derived SSEA-3 negative MSCs (BM non-Muse) as specimens were administered at 3 × 10⁴ cells/1 mL to the produced myocardial infarction model rat through the tail vein of the model rat on the day following model production (day 0). One mL of saline as a medium was administered to the control group. The effect on myocardial infarction was evaluated by echocardiography on day 14 and day 28 after specimen administration.

The evaluation by echocardiography was made by fixing the model rat at the supine position or lateral position under anesthesia with inhalation of 0.5 to 3.0% isoflurane (isoflurane inhalation anesthetic solution "Pfizer" , Mylan Seiyaku Ltd.), and then placing a probe (linear probe or sector probe: 5 to 13 MHz) on the chest of the rat with a universal ultrasonic diagnostic imaging apparatus (Vivid S6, GE Medical Systems) or an ultrasonic diagnostic imaging apparatus (Nemio SSA-550A, Toshiba Medical Systems Corporation) to create an image of the left ventricular short axis plane and measure the left ventricular end-diastolic diameter (LVIDd), the left ventricular end-systolic diameter (LVIDs), the left ventricular anterior wall end-diastolic thickness (LVAWd), the left ventricular free wall end-diastolic thickness (LVPWd), the left ventricular fractional shortening (%FS) and the ejection fraction (EF) at M-mode. These were measured for three heart beats, and the respective average values were defined as measurement values.

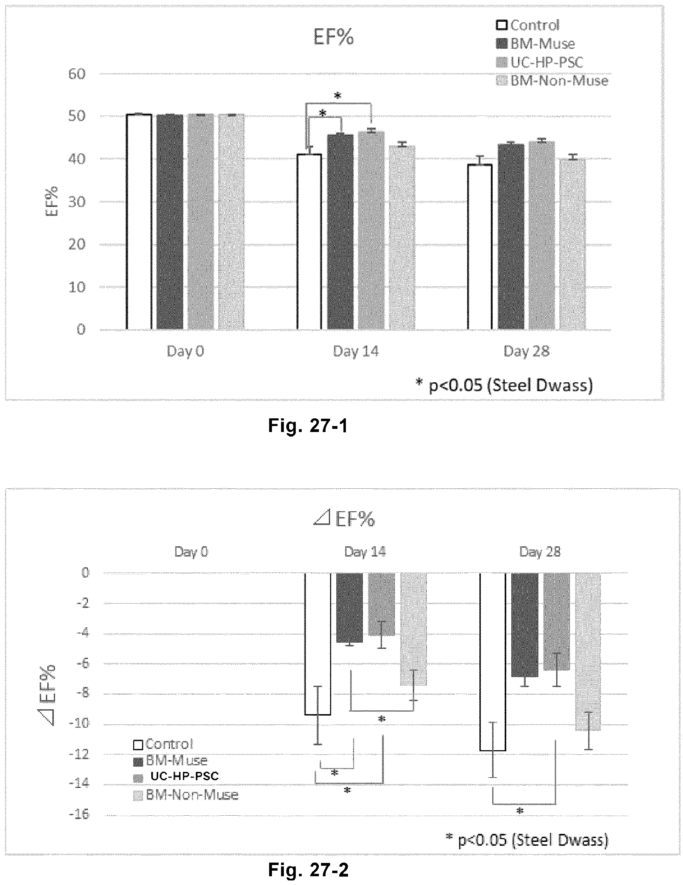

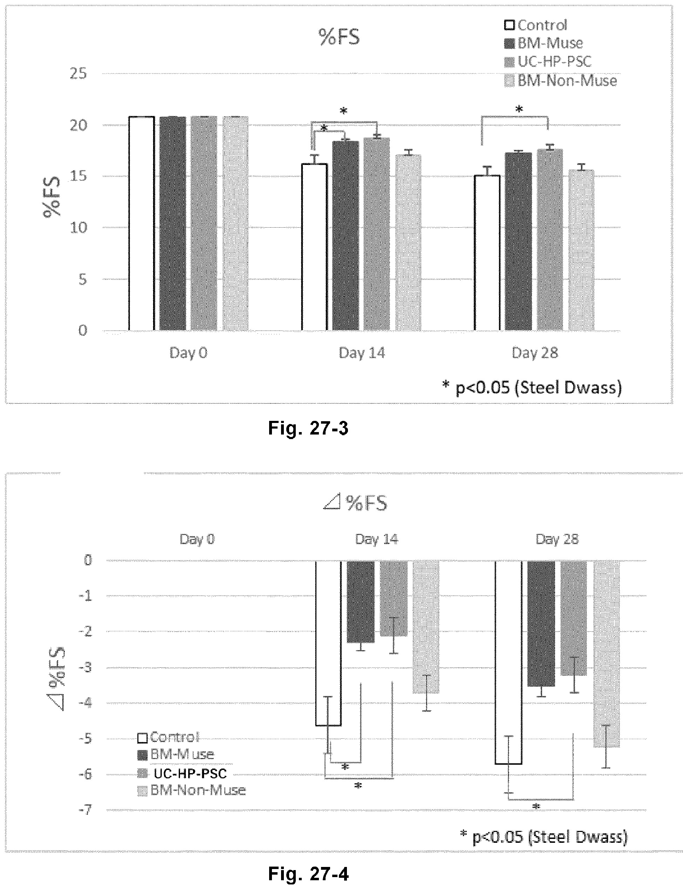

The respective results of EF and %FS are shown in FIGS. 27-1 to 27-4. EF and %FS on day 14 after administration exhibited significantly high values in the BM-Muse group and the UC-HP-PSC group, as compared with those in the control group. %FS on day 28 after administration exhibited a significantly high value in the UC-HP-PSC group, as compared with that in the control group. In this regard, no significant difference was observed at both day 14 and day 28 after administration in the BM non-Muse group, as compared with that in the control group. No significant differences in LVIDs, LVAWd and LVPWd were observed among the groups.

It was thus found from the present Examples that, while both BM-Muse and UC-HP-PSC exhibited effectiveness in the rat myocardial infarction model, UC-HP-PSC exerted a significant effect of %FS even on day 28 after administration and thus had an excellent effect as compared with BM-Muse.

### Example 6: Human umbilical cord tissue-derived high-potential pluripotent stem cells allow for expression of desmosome-associated molecule

Desmosome is an intercellular adhesion device, and is configured from cell membrane proteins such as desmoglein and desmocollin, and intracellular components such as plakoglobin. It is known that desmosome is expressed mainly in epithelial and myocardial cells, and it is reported that desmosome is also expressed in early embryo in normal development and pluripotent stem cells and a desmosome-associated molecule (in particular, desmoglein2 or the like) is a molecule in a human pluripotent stem cell, essential for retention of pluripoteny, and self-renewal (Jongjin Park et al., DSG2 Is a Functional Cell Surface Marker for Identification and Isolation of Human Pluripotent Stem Cells. Stem cell Reports, 11, 115-127 (2018)).

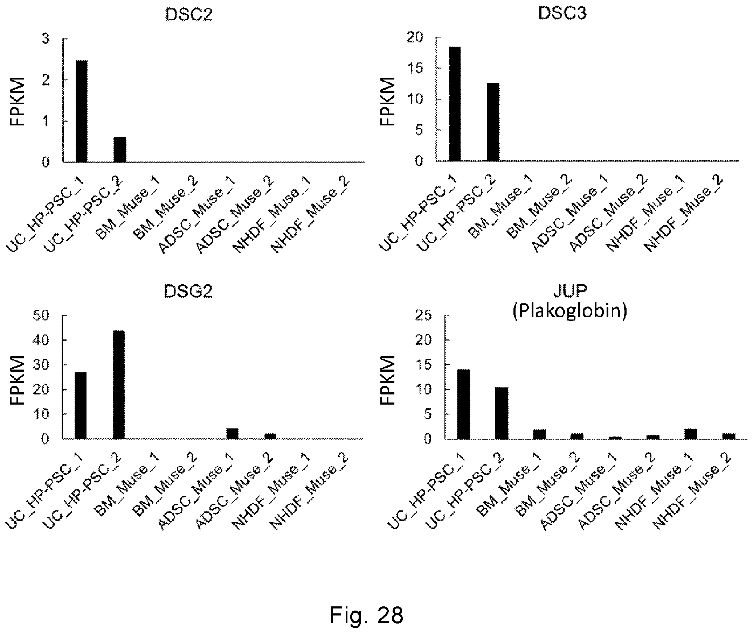

The human umbilical cord-derived high-potential pluripotent stem cells obtained in Example 1-1 were used for exhaustive gene expression analysis with RNA-seq, and expression of desmosome-associated molecules was examined. Bone marrow-, adipose-, and skin-derived Muse cells were used in comparative groups. As a result, high expression of desmoglein2, desmocollin2, desmocollin3, and plakoglobin as desmosome-associated molecules was observed in the human umbilical cord-derived high-potential pluripotent stem cells, as compared with that in bone marrow (BM)-, adipose (ADSC)-, and skin (NHDF)-derived Muse cells, as shown in FIG. 28.

From the foregoing, the high-potential pluripotent stem cells of the present invention is suggested to retain an excellent pluripotent function and have a self-renewal function, as compared with conventional bone marrow-, adipose-, and skin-derived Muse cells, and can also be used in a useful regenerative therapy drug because of resulting in improvement or restoring of a more excellent function of the damaged sites, against various diseases causing tissue damage, and furthermore of being capable of further differentiating into germline cells.

### INDUSTRIAL APPLICABILITY

The high-potential pluripotent stem cells of the present invention not only have characteristics of conventional Muse cells, namely, are capable of differentiating into any embryonic tissue serving as all cells constituting the body, but also are capable of differentiating into any extraembryonic tissue cell such as placenta and/or germline cells, namely, have differentiation capacities close to totipotency. Thus, the high-potential pluripotent stem cells not only allow for conventional regenerative therapies of various diseases which cause damage of tissue constituting the body, but also are capable of differentiating into extraembryonic tissue cells of placenta, uterus, and the like, and thus can allow any damaged sites in such extraembryonic tissues to be re-constituted, resulting in improvement or restoring of the function of such any damaged sites. The high-potential pluripotent stem cells are capable of differentiating into germline cells, and thus can also be used in reproductive therapies such as fertility therapy.

Therefore, the high-potential pluripotent stem cells of the present invention and the cell product comprising the high-potential pluripotent stem cells of the present invention can be applied to a new field of regenerative therapy.

## Claims

1. SSEA-3-positive high-potential pluripotent stem cells.

2. The SSEA-3-positive high-potential pluripotent stem cells according to claim 1, wherein the pluripotent stem cells are derived from an extraembryonic tissue.

3. The SSEA-3-positive high-potential pluripotent stem cells according to claim 2, wherein the extraembryonic tissues are selected from the group consisting of umbilical cord, cord blood, placenta, placenta blood, decidua, chorion, amnion, amniotic fluid, and the like.

4. The SSEA-3-positive high-potential pluripotent stem cells according to any one of claims 1 to 3, wherein the pluripotent stem cell is CD133-positive.

5. The SSEA-3-positive high-potential pluripotent stem cells according to any one of claims 1 to 4, wherein the pluripotent stem cells have differentiation capacities into any extraembryonic tissue cells and/or germline cells.

6. The SSEA-3-positive high-potential pluripotent stem cells according to any one of claims 1 to 5, wherein the pluripotent stem cells have at least one of the following characteristics:
(i) having low or no telomerase activity;
(ii) capable of differentiating into any of triploblastic-lineage cells as having three germ layers;
(iii) showing non-tumorigenic proliferation; and
(iv) having self-renewal capacities.

7. The SSEA-3-positive high-potential pluripotent stem cells according to any one of claims 1 to 6, wherein the pluripotent stem cells have all of the following characteristics:
(i) having low or no telomerase activity;
(ii) capable of differentiating into any of triploblastic-lineage cells as having three germ layers;
(iii) showing non-tumorigenic proliferation; and
(iv) having self-renewal capacities.

8. A regenerative medicine comprising the SSEA-3-positive high-potential pluripotent stem cells according to any one of claims 1 to 7.
